# EUROPÄISCHE PATENTSCHRIFT

(11) **EP 0 373 423 B1**
(45) Veröffentlichungstag und Bekanntmachung des Hinweises auf die Patenterteilung: **12.01.1994**
(21) Anmeldenummer: 89122067.5
(22) Anmeldetag: 29.11.1989
(51) Int. Cl.: C07D 213/64, C07D 213/70, C07F 7/18, A61K 31/44, A61K 31/695

(54) **Substituierte 2-Pyridone und Pyrid-2-thione, Verfahren zu ihrer Herstellung und ihre Verwendung in Arzneimitteln**
Substituted 2-pyridones and pyride-2-thiones, process for their preparation and their use in medicine
Pyridone-2 et pyridinethione-2 substituées, procédé pour leur préparation et leur application comme médicaments

(30) Priorität: 14.12.1988 DE 3841991; 13.06.1989 IT 2086189
(43) Veröffentlichungstag der Anmeldung: 20.06.1990
(73) Patentinhaber: BAYER AG, 51368 Leverkusen (DE)
(72) Erfinder: Fey, Peter, Dr., D-5600 Wuppertal 1 (DE); Angerbauer, Rolf, Dr., D-5600 Wuppertal 1 (DE); Hübsch, Walter, Dr., D-5600 Wuppertal 1 (DE); Philipps, Thomas, Dr., D-5000 Köln 80 (DE); Bischoff, Hilmar, Dr., D-5600 Wuppertal 1 (DE); Petzinna, Dieter, Dr., D-4000 Düsseldorf 12 (DE); Schmidt, Delf, Dr., D-5600 Wuppertal 1 (DE); Thomas, Günter, Dr., I-20020 Arese (Mi) (IT)

(56) Entgegenhaltungen:
- CH-A- 549 569
- CHEMICAL ABSTRACTS, Band 104, Nr. 15, 14. April 1986, Columbus, Ohio, USA H.WEBER et al. "Decker oxidation of 2-substituted N-alkylpyridinium compounds" Seite 689, Spalte 1, Zu- sammenfassung-Nr. 129 776c

## Beschreibung

Die Erfindung betrifft neue substituierte 2-Pyridone und Pyrid-2-thione, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl-Coenzym A Reduktase (HMG-CoA-Reduktase) sind [Mevinolin, EP-A 22 478; US 4 231 938]. Darüber hinaus sind auch bestimmte Indolderivate bzw. Pyrazolderivate Inhibitoren der HMG-CoA-Reduktase [EP-A 1 114 027; US-Patent 4 613 610].

Es wurden nun neue substituierte 2-Pyridone und Pyrid-2-thione der allgemeinen Formeln (I a,b) 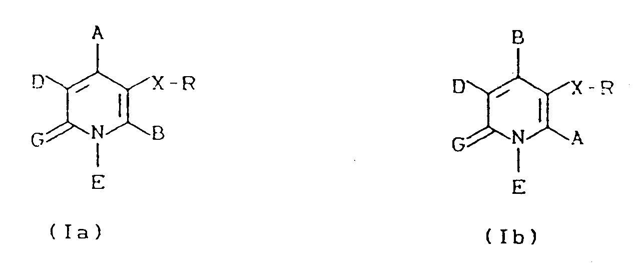
in welcher
- A: - für Oxiranyl, Thienyl, Furyl, Pyridyl, Pyrimidyl, Pyrazinyl, Indolyl, Chinolyl, Isochinolyl, Benzothiazolyl oder Benzimidazolyl steht, das gegebenenfalls bis zu 4-fach gleich oder verschieden durch Fluor, Chlor, Brom, Trifluormethyl, Trifluormethylthio, Trifluormethoxy, geradkettiges oder verzweigtes Alkyl, Alkylthio, Alkylsulfonyl, Alkoxy oder Alkoxycarbonyl mit jeweils bis zu 6 Kohlenstoffatomen, Phenyl, Phenylthio, Phenylsulfonyl oder durch eine Gruppe der Formel -NR¹R² substituiert ist,
worin
- R¹ und R²: gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel -COR³ bedeuten,
worin
- R³: - geradkettiges oder verzweigtes Alkyl oder Alkoxy mit bis zu 6 Kohlenstoffatomen oder Phenyl bedeutet,
oder
- R¹ und R²: gemeinsam mit dem Stickstoffatom einen 5- bis 7-gliedrigen Ring bilden, der durch geradkettiges oder verzweigtes Alkyl mit bis zu 6 Kohlenstoffatomen substituiert sein kann,
- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy, Alkoxy mit bis zu 4 Kohlenstoffatomen, Phenyl oder durch eine Gruppe der Formel -NR¹R² substituiert sein können, oder durch Phenyl, Phenyloxy, Phenylthio, Phenylsulfonyl, Fluor, Chlor, Brom, Nitro, Cyano, Trifluormethyl, Trifluormethoxy, Trifluormethylthio, Benzyloxy oder durch eine Gruppe der Formel -NR¹R² substituiert ist,
- B: - für Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, Cyano, Azido, Trifluormethyl, Trifluormethoxy, Trifluormethylthio, Trifluormethylsulfonyl, Alkoxy mit bis zu 8 Kohlenstoffatomen oder durch Phenyl, Phenyloxy, oder Phenylthio, Thienyl, Furyl, Pyridyl, Pyrimidyl oder Chinolyl substituiert ist, welches seinerseits bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein kann, oder
   durch eine Gruppe der Formel -NR¹R² oder -COR³ substituiert ist
- für Phenyl steht, das gegebenenfalls bis zu 2-fach gleich oder verschieden durch Fluor, Chlor, Brom, Cyano, Nitro, Trifluormethyl, geradkettiges oder verzweigtes Alkyl, Alkoxy oder Alkoxycarbonyl mit jeweils bis zu 6 Kohlenstoffatomen oder Amino substituiert ist,
- D und E: gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclobutyl, Cyclopentyl oder Cyclohexyl stehen,
- für geradkettiges oder verzweigtes Alkyl oder Alkenyl mit jeweils bis zu 10 Kohlenstoffatomen oder Imino stehen, die gegebenenfalls durch Fluor, Chlor, Brom, Azido, 2,5-Dioxotetrahydropyrryl, Phenyl, Pyrimidyl, Pyrryl, Pyrrolidinyl, Morpholino oder Morpholino-N-oxid substituiert ist, oder durch eine Gruppe der Formel NR¹R², -OR⁴, -COR⁵ oder -S(O)ₙ-R⁶ substituiert sind,
worin
- R¹ und R²: die oben angegebene Bedeutung haben,
- R⁴: - Wasserstoff oder
- geradkettiges oder verzweigtes Alkyl mit bis zu 8 Kohlenstoffatomen bedeutet, das gegebenefalls durch Hydroxy, Trialkylsilyl mit bis zu 8 Kohlenstoffatomen im gesamten Alkylteil, Fluor, Chlor, Brom oder durch Phenyl substituiert ist, welches seinerseits durch Fluor, Chlor, Brom, Cyano, Nitro, Hydroxy, geradkettiges oder verzweigtes Alkyl, Alkoxy oder Alkoxycarbonyl mit jeweils bis zu 6 Kohlenstoffatomen oder Amino substituiert sein kann,
- Trialkylsilyl mit bis zu 8 Kohlenstoffatomen im gesamten Alkylteil, Tetrahydropyranyl oder 2,5-Dioxo-tetrahydropyrryl bedeutet,
- Cyclopropyl, Cyclobutyl, Cyclopentyl, Cyclohexyl oder Phenyl bedeutet, welches seinerseits durch Fluor, Chlor, Brom, Cyano, Nitro oder Amino substituiert sein kann, oder
- eine Gruppe der Formel -COR⁷ bedeutet,
worin
- R⁷: - geradkettiges oder verzweigtes Alkyl mit bis zu 6 Kohlenstoffatomen, Phenyl oder eine Gruppe der Formel -NR¹R² bedeutet,
- R⁵: - Wasserstoff, geradkettiges oder verzweigtes Alkyl mit bis zu 8 Kohlenstoffatomen bedeutet, das gegebenenfalls durch Hydroxy, Phenyl, Fluor, Chlor, Brom oder Cyano substituiert ist,
- Phenyl, Naphthyl, Pyrryl, Pyrimidyl, Pyridyl, Pyrrolidinyl oder Morpholino bedeutet, welches seinerseits durch Fluor, Chlor, Brom, Amino, Hydroxy, Nitro oder Cyano substituiert sein kann, oder
- eine Gruppe der Formel -NR¹R² oder -OR⁴ bedeutet,
- n: - eine Zahl 0 oder 2 bedeutet,
- R⁶: - geradkettiges oder verzweigtes Alkyl mit bis zu 8 Kohlenstoffatomen bedeutet, das durch Fluor, Chlor, Brom, Hydroxy, Phenyl oder durch eine Gruppe der Formel -NR¹R² substituiert sein kann,
- Phenyl bedeutet, das durch Fluor, Chlor, Brom, Hydroxy, Cyano, Nitro oder Amino substituiert sein kann, oder
- eine Gruppe der Formel -NR¹R² bedeutet, wenn n für die Zahl 2 steht,
oder
- D und E: gleich oder verschieden sind und
- für eine Gruppe der Formel -NR¹R², -OR⁴ oder -COR⁵ stehen,
worin
- R¹, R², R⁴ und R⁵: die oben angegebene Bedeutung haben,
- D oder E: gemeinsam mit B einen 5- bis 7-gliedrigen, gesättigten oder ungesättigten Ring bilden, der gegebenenfalls durch geradkettiges oder verzweigtes Alkyl mit bis zu 6 Kohlenstoffatomen oder Phenyl substituiert ist,
- G: - für ein Sauerstoff- oder ein Schwefelatom steht,
- X: - für eine Gruppe der Formel -CH₂-CH₂- oder -CH=CH-steht
und
- R: - für eine Gruppe der Formel 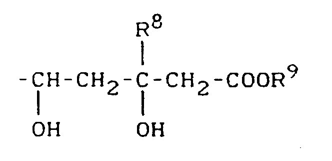 oder 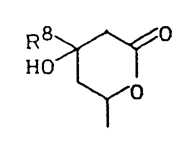 steht,
worin
- R⁸: - Wasserstoff
- R⁹: - Wasserstoff oder geradkettiges oder verzweigtes Alkyl mit bis zu 8 Kohlenstoffatomen oder Benzyl bedeutet, oder
- Phenyl oder ein Kation bedeutet,
oder
- D: - für die Gruppe der Formel -X-R steht,
worin
- X und R: die oben angegebene Bedeutung haben
und deren Salze gefunden.

Bildet R⁹ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester (C₁ bis C₆) und Aralkylester (C₇ bis C₁₀), bevorzugt (C₁-C₄)-Alkylester sowie Benzylester. Darüberhinaus seien die folgenden Esterreste genannt: Methylester, Ethylester, Propylester, Benzylester.

Steht R⁹ für ein Kation, so ist bevorzugt ein physiologisch verträgliches Metall- oder Ammoniumkation gemeint. Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie (C₁-C₄)-Dialkylamine, (C₁-C₄)-Trialkylamine, Prokain, Dibenzylamin, N,N'-Dibenzylethylendiamin, N-Benzyl-β-phenylethylamin, N-Methylmorpholin oder N-Ethylmorpholin, 1-Ephenamin, Dihydroabietylamin, N,N'-Bis-dihydroabietylethylendiamin, N-Niederalkylpiperidin und andere Amine, die zur Bildung von Salzen verwendet werden können.

Überraschenderweise zeigen die erfindungsgemäßen substituierten 2-Pyridone und Pyrid-2-thione eine überlegene inhibitorische Wirkung auf die HMG-CoA-Reduktase (3-Hydroxy-3-methyl-glutaryl-Coenzym A Reduktase).

Bevorzugt sind Verbindungen der allgemeinen Formeln (Ia) und (Ib),
in welchen
- A: - für Oxiranyl, Pyridyl, Pyrimldyl, Chinolyl oder Isochinolyl steht, das gegebenenfalls bis zu 3-fach gleich oder verschieden durch Fluor, Chlor, Trifluormethyl, geradkettiges oder verzweigtes Alkyl, Alkylthio, Alkylsulfonyl, Alkoxy oder Alkoxycarbonyl mit jeweils bis zu 4 Kohlenstoffatomen, Phenyl, Phenylthio, Phenylsulfonyl oder durch eine Gruppe der Formel -NR¹R² substituiert ist,
worin
- R¹ und R²: gleich oder verschieden sind und
- Wasserstoff, Phenyl, Phenylsulfonyl, geradkettiges oder verzweigtes Alkyl oder Alkylsulfonyl mit bis zu 4 Kohlenstoffatomen, Benzyl oder Benzylsulfonyl bedeuten,
- eine Gruppe der Formel -COR³ bedeutet,
worin
- R³: - geradkettiges oder verzweigtes Alkyl oder Alkoxy mit bis zu 4 Kohlenstoffatomen oder Phenyl bedeutet,
oder
- R¹ und R²: gemeinsam mit dem Stickstoffatom einen 5-bis 7-gliedrigen Ring bilden, der durch geradkettiges oder verzweigtes Alkyl mit bis zu 4 Kohlenstoffatomen substituiert sein kann,
- für Phenyl steht, das gegebenenfalls bis zu 3-fach gleich oder verschieden substituiert ist durch geradkettiges oder verzweigtes Alkyl, Alkylthio, Alkylsulfonyl, Alkoxy oder Alkoxycarbonyl mit jeweils bis zu 6 Kohlenstoffatomen, welche ihrerseits durch Hydroxy, Methoxy, Ethoxy, Propoxy, Phenyl oder durch eine Gruppe der Formel -NR¹R² substituiert sein können,
   oder durch Phenyl, Phenyloxy, Fluor, Chlor, Nitro, Cyano, Trifluormethyl, Benzyloxy oder eine Gruppe der Formel -NR¹R² substituiert ist,
worin
- R¹ und R²: die oben angegebene Bedeutung haben,
- B: - für Cyclopropyl, Cyclobutyl, Cyclopentyl oder Cyclohexyl steht,
- für geradkettiges oder verzweigtes Alkyl mit bis zu 8 Kohlenstoffatomen steht, das gegebenenfalls durch Fluor, Chlor, Cyano, Azido, Alkoxy mit bis zu 6 Kohlenstoffatomen, Phenyl oder Phenoxy substituiert ist, die ihrerseits durch Fluor, Chlor, Cyano, geradkettiges oder verzweigtes Alkyl oder Alkoxy mit bis zu 4 Kohlenstoffatomen oder durch eine Gruppe der Formel -NR¹R² oder -COR³ substituiert sind,
- für Phenyl steht, das durch Fluor, Chlor, Nitro, geradkettiges oder verzweigtes Alkyl, Alkoxy oder Alkoxycarbonyl mit jeweils bis zu 4 Kohlenstoffatomen oder Amino substituiert ist,
- D und E: gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclopentyl oder Cyclohexyl stehen,
- für geradkettiges oder verzweigtes Alkyl oder Alkenyl mit jeweils bis zu 8 Kohlenstoffatomen oder Imino stehen, die gegebenenfalls durch Fluor, Chlor, Azido, 2,5-Dioxo-tetrahydropyrryl, Phenyl, Pyrrolidinyl, Morpholino oder Morpholino-N-oxid substituiert ist, oder durch eine Gruppe der Formel -NR¹R², -OR⁴, -COR⁵ oder -S(O)ₙ-R⁶ substituiert sind,
worin
- R¹ und R²: die oben angegebene Bedeutung haben,
- R⁴: - Wasserstoff oder
- geradkettiges oder verzweigtes Alkyl mit bis zu 6 Kohlenstoffatomen bedeutet, das gegebenenfalls durch Hydroxy, Dimethyl-tert.-butylsilyl, Fluor, Chlor oder durch Phenyl substituiert ist, welches seinerseits durch Fluor, Chlor, Hydroxy oder Amino substituiert sein kann,
- Trialkylsilyl mit bis zu 6 Kohlenstoffatomen im gesamten Alkylteil, Tetrahydropyranyl oder 2,5-Dioxo-tetra-hydropyrryl bedeutet,
- Cyclopropyl, Cyclopentyl, Cyclohexyl oder Phenyl bedeutet, oder
- eine Gruppe der Formel -COR⁷ bedeutet,
worin
- R⁷: - geradkettiges oder verzweigtes Alkyl mit bis zu 4 Kohlenstoffatomen, Phenyl oder eine Gruppe der Formel -NR¹R² bedeutet,
- R⁵: - Wasserstoff, geradkettiges oder verzweigtes Alkyl mit bis zu 6 Kohlenstoffatomen bedeutet, das gegebenenfalls durch Hydroxy, Phenyl, Fluor oder Chlor substituiert ist,
- Phenyl, Pyrryl, Pyrrolidinyl oder Morpholino bedeutet, oder
- eine Gruppe der Formel -NR¹R² oder -OR⁴ bedeutet,
- n: - eine Zahl 0 oder 2 bedeutet,
- R⁶: - geradkettiges oder verzweigtes Alkyl mit bis zu 6 Kohlenstoffatomen bedeutet, das durch Fluor, Chlor, Hydroxy, Phenyl oder durch eine Gruppe der Formel -NR¹R² substituiert sein kann,
- Phenyl bedeutet, das durch Fluor, Chlor, Hydroxy, Cyano, Nitro oder Amino substituiert sein kann,
- eine Gruppe der Formel -NR¹R² bedeutet, wenn n für die Zahl 2 steht,
oder
- D und E: gleich oder verschieden sind und
- für eine Gruppe der Formel -NR¹R², -OR⁴ oder -COR⁵ stehen,
worin
- R¹, R², R⁴ und R⁵: die oben angegebene Bedeutung haben,
oder
- D oder E: gemeinsam mit B einen 5- bis 7-gliedrigen, gesättigten oder ungesättigten Ring bilden, der gegebenenfalls durch Methyl, Ethyl, Propyl, Isopropyl, Butyl, tert.Butyl oder Phenyl substituiert ist,
- G: - für ein Sauerstoff- oder Schwefelatom steht,
- X: - für eine Gruppe -CH=CH- steht
und
- R: - für eine Gruppe der Formel 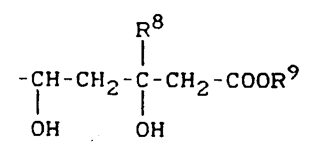 oder 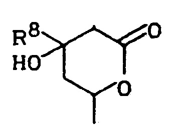 steht,
worin
- R⁸: - Wasserstoff, Methyl, Ethyl, Propyl, Isopropyl, Butyl, Isobutyl oder tert.Butyl bedeutet
und
- R⁹: - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet
oder
- D: - für eine Gruppe der Formel -X-R steht,
worin
- X und R: die oben angegebene Bedeutung haben
und deren Salze.

Die erfindungsgemäßen substituierten 2-Pyridone und Pyrid-2-thione der allgemeinen Formeln (Ia,b) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes R ergeben sich unterschiedliche Stereoisomere, die im folgenden näher erläutert werden sollten:
a) Steht die Gruppe -X- für eine Gruppe der Formel -CH=CH- , so können die erfindungsgemäßen Verbindungen in zwei stereoisomeren Formen existieren, die an der Doppelbindung E-konfiguriert oder Z-konfiguriert sein können:
   Bevorzugt sind diejenigen Verbindungen, die E-konfiguriert sind.
b) Steht der Rest -R- für eine Gruppe der Formel 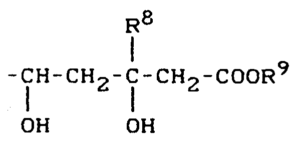 so besitzen die Verbindungen der allgemeinen Formeln (Ia,b) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration oder in der threo-Konfiguration vorliegen.
   Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).
   Bevorzugt sind hierbei die Erythro-konfigurierten Isomeren, besonders bevorzugt das 3R,5S-Isomere sowie das 3R,5S-3S,5R-Racemat.
c) Steht der Rest -R- für eine Gruppe der Formel 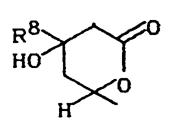 so besitzen die substituierten 2-Pyridone und Pyrid-2-thione mindestens zwei asymmetrische Kohlenstoffatome, nämlich das Kohlenstoffatom an dem die Hydroxygruppe gebunden ist, und das Kohlenstoffatom an welchem der Rest der Formel 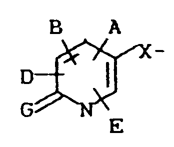 gebunden ist. Je nach der Stellung der Hydroxygruppe zur freien Valenz am Lactonring können die substituierten 2-Pyridone und Pyrid-2-thione als cis-Lactone oder als trans-Lactone vorliegen.

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomeren sind die trans-Lactone. Besonders bevorzugt ist hierbei das 4R,6S-Isomere (trans) sowie das 4R,6S-4S,6R-Racemat.

Außerdem wurde ein Verfahren zur Herstellung der substituierten 2-Pyridone und Pyrid-2-thione der allgemeinen Formeln (I a,b) 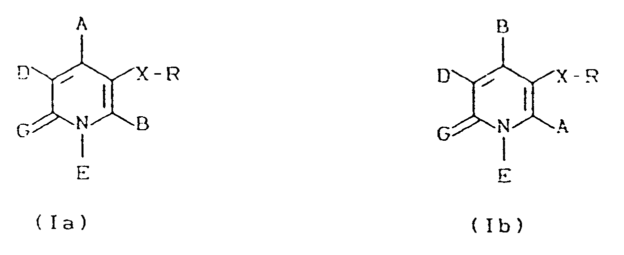
in welcher
A, B, D, E, G, X und R die oben angegebene Bedeutung haben,
gefunden, das dadurch gekennzeichnet ist, daß man
Ketone der allgemeinen Formel (VIII) 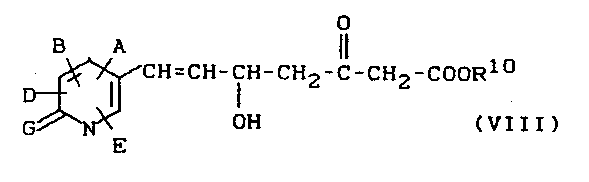
in welcher
A, B, D, E und G die oben angegebene Bedeutung haben,
und
R¹⁰ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = -CH₂-CH₂-) die Ethenverbindungen (X = -CH=CH-) nach üblichen Methoden hydriert,
und gegebenenfalls Isomeren trennt.

Das erfindungsgemäße Verfahren kann durch das folgende Formelschema erläutert werden: 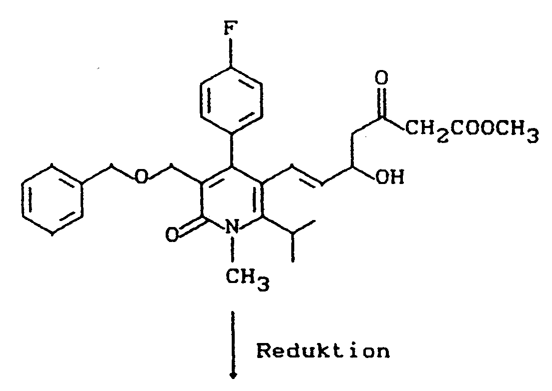 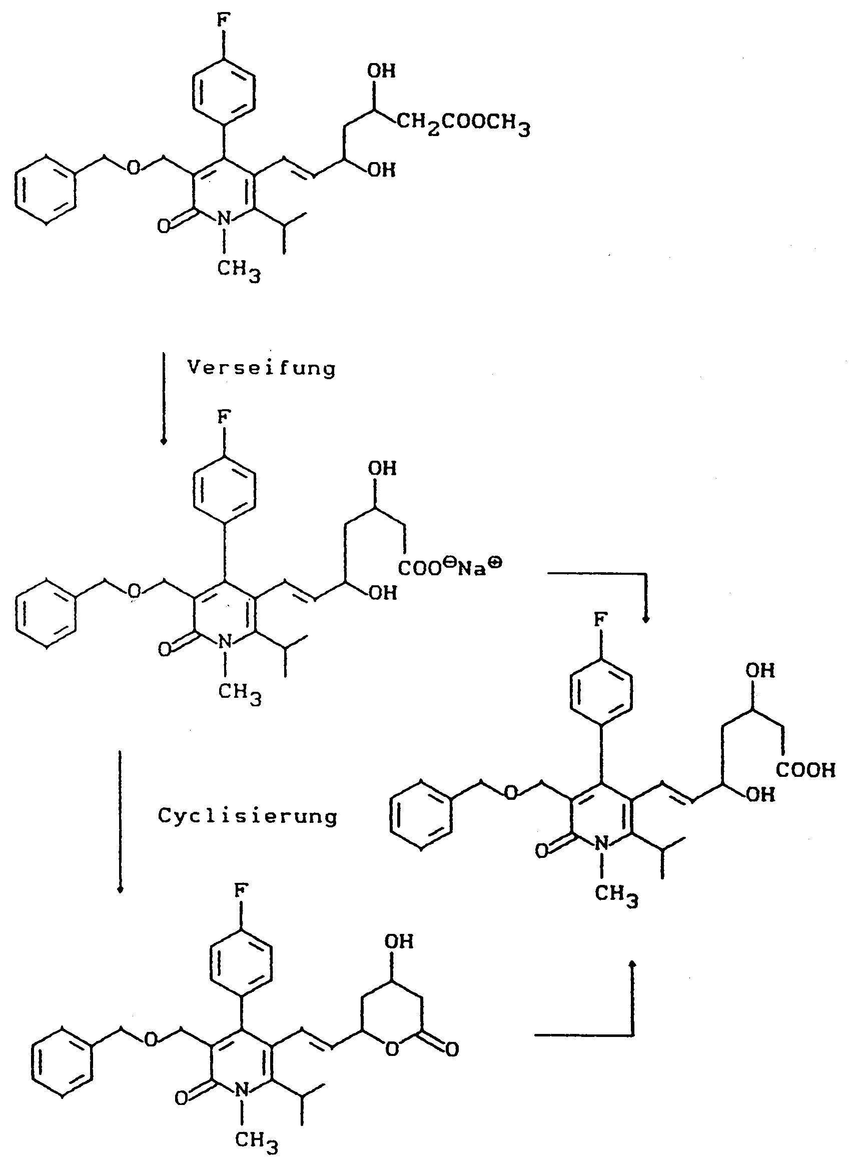
Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern, Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungesgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formeln (Ia,b), in welchen X für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formeln (Ia,b) entsprechen der Formel (Ic) 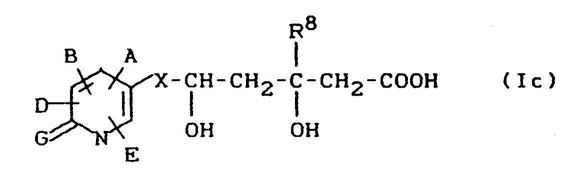
in welcher
A, B, D, E, G und R⁸ die oben angegebene Bedeutung haben.

Die Carbonsäureester im Rahmen der allgemeinen Formeln (Ia,b)entsprechen der Formel (Id) 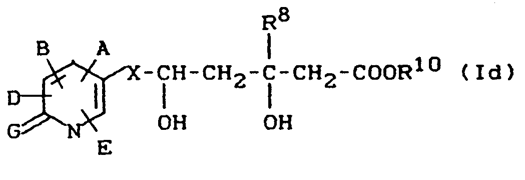
in welcher
A, B, D, E, G und R⁸ die oben angegebene Bedeutung haben,
und
R¹⁰ - für Alkyl steht.

Die Salze der erfindungsgemäßen Verbindungen im Rahmen der allgemeinen Formeln (Ia,b)entsprechen der Formel (Ie) 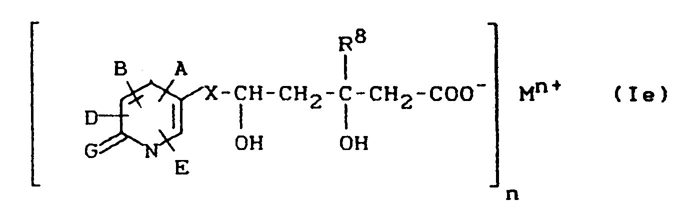
in welcher
A, B, D, E, G und R⁸ die oben angegebene Bedeutung haben,
und
Mⁿ⁺ für ein Kation steht, wobei n die Wertigkeit angibt.

Die Lactone im Rahmen der allgemeinen Formeln (Ia,b)entsprechen der Formel (If) 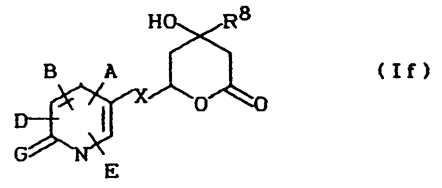
in welcher
A, B, D, E, G und R⁸ die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-paratoluolsulfonat, N-Cyclohexyl-N′-[2-(N˝-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Ganz besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig) 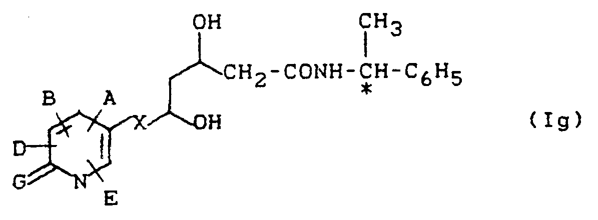
überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden: 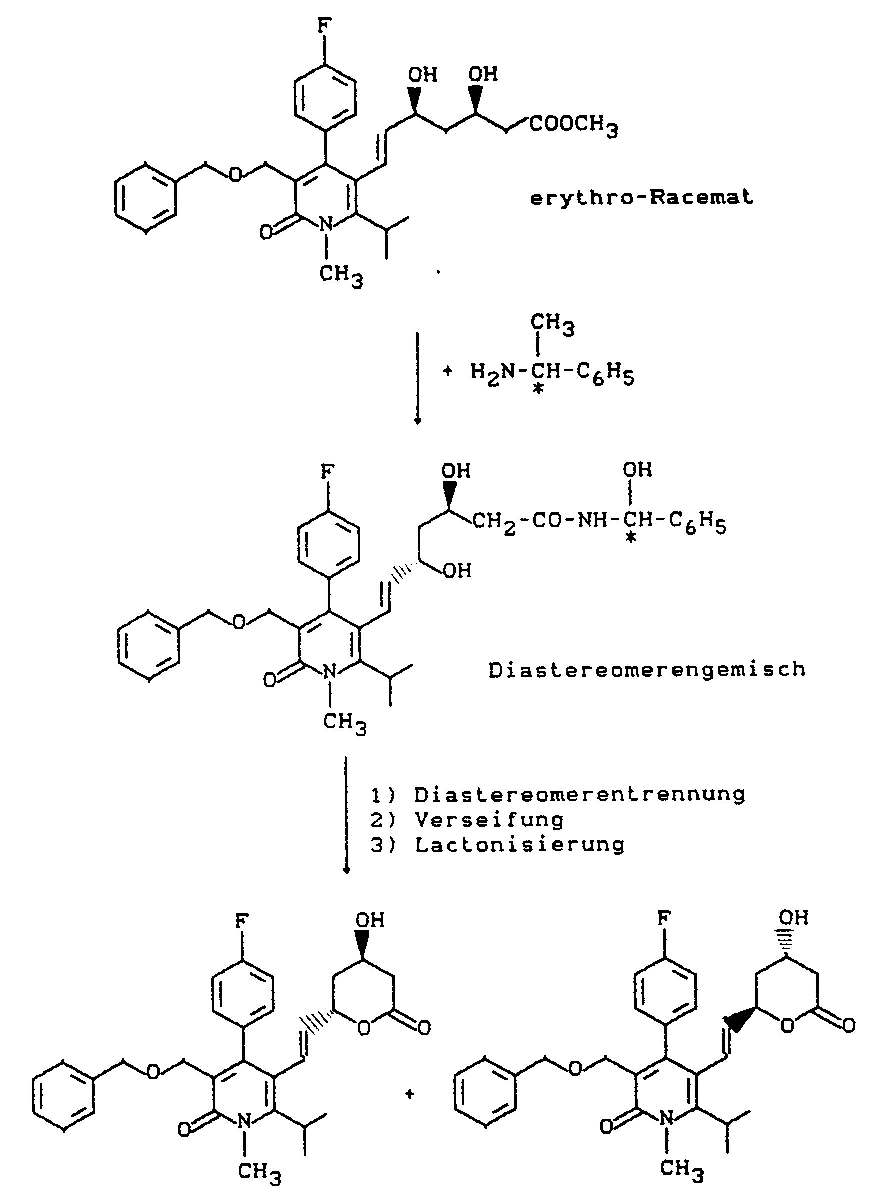
Die als Ausgangsstoffe eingesetzten Ketone (VIII) sind neu.

Es wurde ein Verfahren zur Herstellung der erfindungsgemäßen Ketone der allgemeinen Formel (VIII) 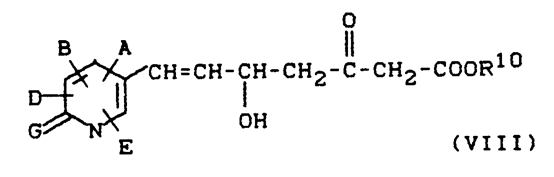
in welcher
A, B, D, E, G und R¹⁰ die oben angegebene Bedeutung haben,
gefunden, das dadurch gekennzeichnet ist, daß man
Aldehyde der allgemeinen Formel (IX) 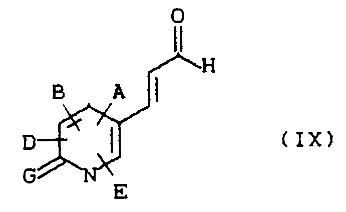
in welcher
A, B, D, E und G die oben angegebene Bedeutung haben,
in inerten Lösemitteln mit Acetessigester der allgemeinen Formel (X) 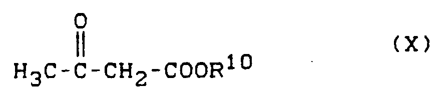
in welcher
R¹⁰ die oben angegebene Bedeutung hat,
in Anwesenheit von Basen umsetzt.

Das erfindungsgemäße Verfahren kann beispielsweise durch folgendes Formelschema erläutert werden: 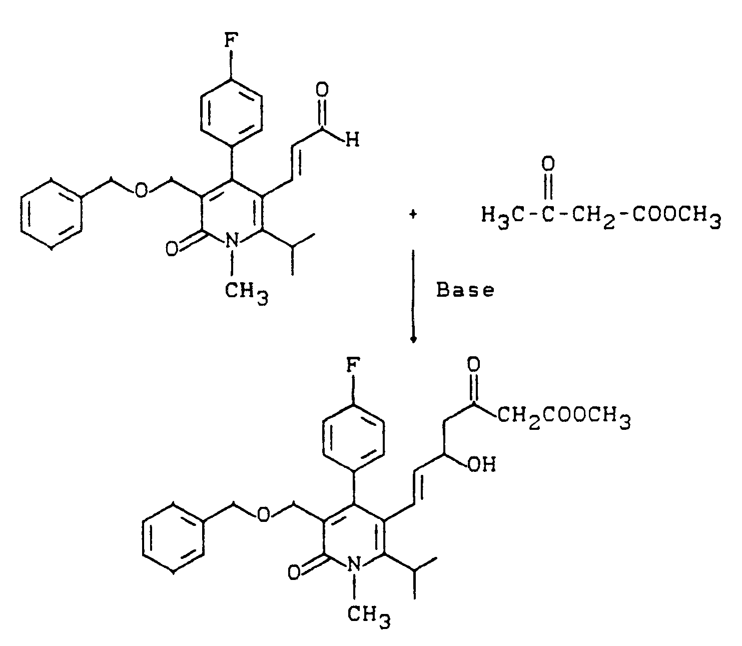
Als Basen kommen hierbei die üblichen stark basischen Verbindungen in Frage. Hierzu gehören bevorzugt lithiumorganische Verbindungen wie beispielsweise n-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden n-Butyllithium oder Natriumhydrid oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die 2-Pyridone des Typs (Ia) erläutert werden. 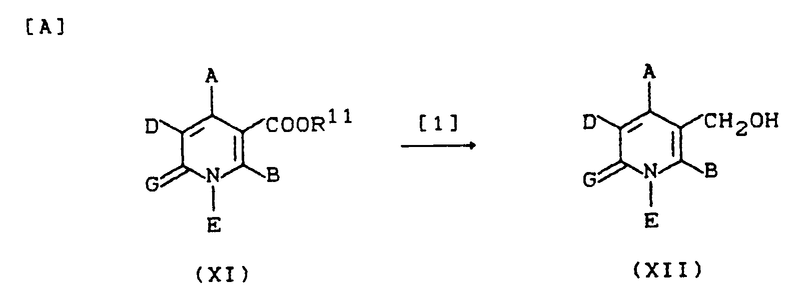 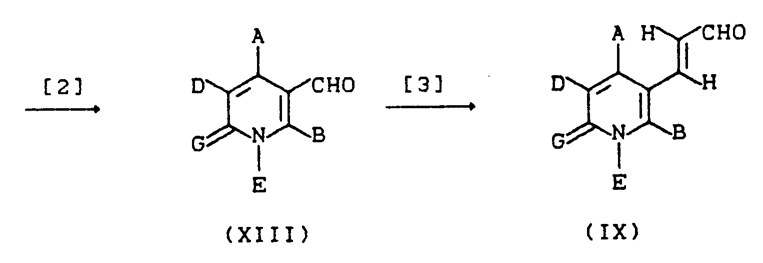
Hierbei werden gemäß Schema A 2-Pyridone der Formel (XI),in welchen R¹¹ für einen Alkylrest mit bis zu 4 Kohlenstoffatomen steht, im ersten Schritt [1] in inerten Lösemitteln wie Ethern, beispielsweise Diethylether, Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70°C bis +100°C, vorzugsweise von -70°C bis Raumtemperatur, bzw. von Raumtemperatur bis +70°C je nach verwendetem Reduktionsmittel zu den Hydroxymethylverbindungen (XII) reduziert. Vorzugsweise wird die Reduktion mit Diisobutylaluminiumhydrid in Tetrahydrofuran in einem Temperaturbereich von -78°C bis Raumtemperatur durchgeführt. Die Hydroxymethylverbindungen (XII) werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XIII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten Pyridone der Formel (XI) sind neu. Man erhält sie im allgemeinen gemäß Schema B durch Oxidation von 3,4-Dihydropyrid-2-onen (XIV). Die Oxidation der Dihydropyridone (XIV) zu den Pyridonen (XI), in welchen R¹¹ die oben angegebene Bedeutung hat, kann beispielsweise mit Chromoxid oder Natriumnitrit in Eisessig in einem Temperaturbereich von -20°C bis +150°C, mit Salpetersäure in wäßriger Suspension oder mit Cersalzen, wie beispielsweise Ammoniumcernitrat, in einem Lösungsmittelgemisch aus Acetonitril und Wasser durchgeführt werden. Vorzugsweise wird mit Ammoniumcernitrat in dem Gemisch Acetonitril und Wasser umgesetzt. 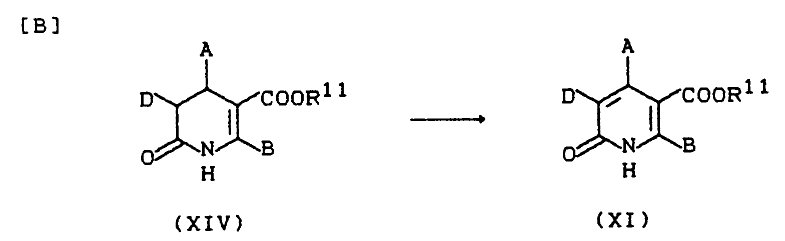
Die hierbei als Ausgangsstoffe eingesetzten 3,4-Dihydropyrid-2-one der allgemeinen Formel (XIV) sind neu.

Man erhält sie im allgemeinen durch Reaktion geeignet substituierter α,β-ungesättigter Carbonsäureester der allgemeinen Formel (XV), worin A, B, D und R¹¹ die oben angegebene Bedeutung haben, und entsprechend substituierten β-Amino-α,β-ungesättigten Carbonsäureestern der allgemeinen Formel (XVI).

Das Verfahren kann in Substanz oder in einem hochsiedendem Lösemittel wie beispielsweise Ethylenglykol entweder basisch mit Alkalialkoholaten, wie beispielsweise Natrium- oder Kaliumethanolat bei Raumtemperatur bis +200°C oder in Eisessig bei Raumtemperatur durchgeführt werden. Bevorzugt ist die Umsetzung mit Alkalialkoholaten bei +140°C.

Die Reaktion kann durch folgendes Formelschema erläutert werden: 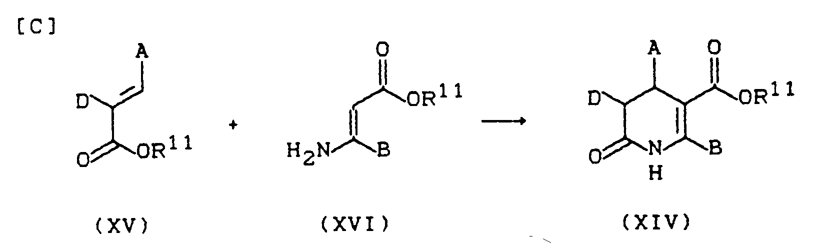
Die Verbindungen der allgemeinen Formel (I), in welcher A, B, D, E, X und R die oben angegebene Bedeutung haben, und G für Schwefel steht, können aus den 2-Pyridonen der allgemeinen Formel (XI), in welcher A, B, D und E die oben angegebene Bedeutung haben, gegebenenfalls nach literaturbekannten Methoden erhalten werden [A.Y. Guttsait et al., Khim. Geterotsikl. Soedin 1987, 9, 1233 - 1237].

Die Pyridone (XI), die wie oben beschrieben, aus den Dihydropyridonen (XIV) durch Oxidation hergestellt werden, können durch geeignete Reduktionsmittel, wie beispielsweise Lithiumaluminiumhydrid, Diisobutylaluminiumhydrid oder Natrium-bis-(2-methoxyethoxy)-dihydroaluminat in inerten Lösemitteln, wie beispielsweise Tetrahydrofuran oder Toluol, zu den Pyridonen (XVIII) reduziert werden.

Die Pyridone (XVIII) können nach bekannten Methoden zu den Pyridonen (XIX) umgesetzt werden, beispielsweise durch Reaktion mit einem Alkyl- oder Benzylhalogenid in Gegenwart einer Base wie beispielsweise Natriumhydrid oder beispielsweise durch Umsetzung mit einem Trialkylsilylhalogenid oder einem Säurehalogenid in Gegenwart einer Base wie Imidazol, Pyridin oder Triethylamin. Die Hydroxygruppe der Pyridone (XVIII) kann nach bekannten Methoden in eine Abgangsgruppe überführt werden, z.B. durch Umsetzung mit Trifluormethansulfonsäureanhydrid, Thionylchlorid oder Methansulfonsäurechlorid in Gegenwart einer Base. Die Abgangsgruppe kann dann nach bekannten Methoden gegen Nucleophile ausgetauscht werden. 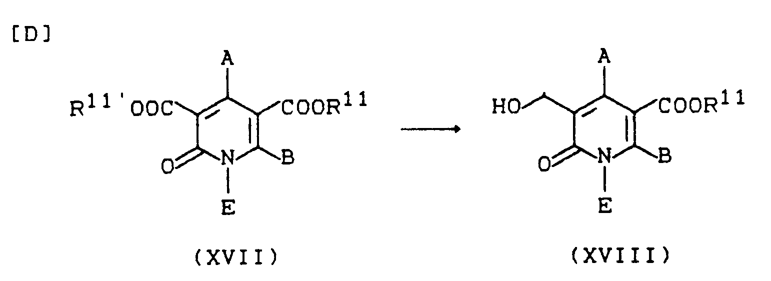 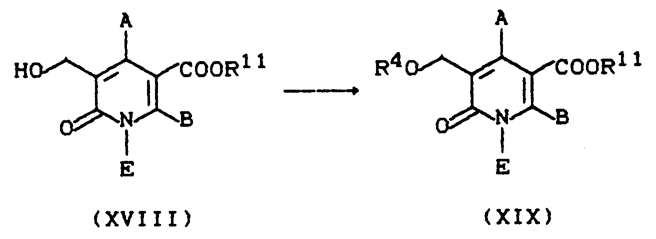
Die Reste A, B, E, R⁴ und R¹¹ der Formeln (XVII), (XVIII) und (XIX) haben die oben angegebene Bedeutung.

Durch Reaktion der Pyridone (XVIII) bzw. (XVII), deren Reste A, B und R¹¹ die oben angegebene Bedeutung haben und E für Wasserstoff steht, mit Alkyl- oder Benzylhalogeniden in Gegenwart einer Base wie beispielsweise Kaliumcarbonat, Natriumhydrid oder einem Säurehalogenid in Gegenwart einer Base wie Imidazol, Pyridin oder Triethylamin können die N-Alkyl bzw. N-Acylderivate hergestellt werden.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methyl-glutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G. C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat, 0,03 m Ethylendiamintetraessigsäure, 0,002 m Dithiothreit (SPE)-Puffer pH 7,2, homogenisiert. Anschließend wurde 15 Minuten bei 15.000 g zentrifugiert und das Sediment verworfen. Der Überstand wurde 75 Minuten bei 100.000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumens SPE-Puffer aufgenommen, homogenisiert und bei -78°C eingefroren und gelagert (= Enzym-lösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol KₓH_{y}-Phosphat pH 7,2, 20 µl Enzympräparation und 56 nMol 3-Hydroxy-3-methyl-glutaryl Coenzym A (Glutaryl-3-¹⁴C) 100.000 dpm.

Nach Inkubation von 60 Minuten bei 37°C wurde der Ansatz zentrifugiert und 600 µl des Überstandes auf eine 0,7 x 4 cm mit einem 5-Chlorid Anionenaustauscher (100-200 mesh) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 1 gesetzt und mit dem simultan bestimmten IC₅₀-Wert der Testverbindung verglichen.

Messung der Chloresterinbiosynthese nach Gabe von HMG-CoA-Reduktase-Inhibitoren.

Männliche Ratten (ca. 180 g) erhalten 16 h nach Futterentzug die Testsubstanz in 10 ml/kg 0,75 %iger Tragantlösung. Die Kontrollgruppe erhält nur das Vehikel. Zu verschiedenen Zeiten nach Substanzapplikation erhalten die Tiere 20 µ Ci ¹⁴C-Acetat pro Tier intraperitoneal. Zu unterschiedlichen Zeiten nach der ¹⁴C-Acetatinjektion werden die Tiere getötet, die Lebern entnommen und nach Extraktion und anschließender Radioaktivitätsmessung die Chloresterinsyntheserate bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle dar Applikation größerer Mengen kann es empfehlenswert sein, diese in mehreren Einzelgaben über den Tag zu verteilen.

### Herstellungbeispiele

### Beispiel 1

### 3-Amino-4-methyl-pent-2-en-säure-ethylester

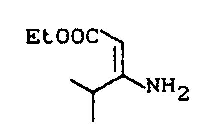

Zu 500 g (3,16 mol) Isobutyrylessigsäureethylester in 1500 ml Toluol p.A. werden 10,8 g p-Toluolsulfonsäure x 4 H₂O gegeben, die Mischung unter Rühren bei Raumtemperatur mit Ammoniak-Gas gesättigt und über Nacht stehen gelassen. Anschließend wird am Wasserabscheider unter Rückfluß erhitzt und kontinuierlich Ammoniak-Gas eingeleitet, bis die berechnete Wassermenge abgeschieden ist (nach 8 Stunden Rückfluß 47 ml Wasser). Man läßt über Nacht abkühlen, saugt den ausgefallenen Niederschlag ab und wäscht mit Toluol nach. Die vereinigten Toluolphasen werden mehrfach mit Wasser gewaschen, mit Natriumsulfat getrocknet, im Vakuum eingeengt und im Hochvakuum destilliert.
Kp.: 82 - 85°C / 1 Torr.
Ausbeute: 315 g (63,4% der Theorie, ca. 90%ig).
- ¹H-NMR (CDCl₃):: δ (ppm) = 1,13 (d, 6H); 1,25 (t, 3H); 2,32 (sept., 1H); 4,12 (q, 2H); 4,56 (s, 1H).

### Beispiel 2

### 1-Carbomethoxy -2-(4-fluorphenyl)-propensäuremethylester

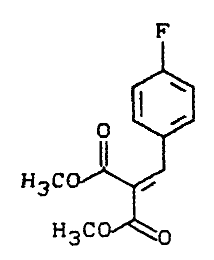

229 ml (2 mol) Malonsäuredimethylester, 223 ml (2 mol) 4-Fluorbenzaldehyd, 40 ml Piperidin und 103 ml Eisessig werden in 1,5 l Cyclohexan über Nacht am Wasserabscheider unter Rückfluß erhitzt. Nach Abkühlen auf Raumtemperatur wird in Essigester aufgenommen, mit Wasser gewaschen, mit Natriumsulfat getrocknet und destilliert.
Sdp.: 135°C - 140°C (1 mm)
Ausbeute: 342,9 g (72% der Theorie)
- ¹H-NMR (CDCl₃):: δ (ppm) = 3,85 (s, 6H); 7,0 - 7,5 (m, 4H); 7,7 (s, 1H).

### Beispiel 3

### 3,4-Dihydro-4-(4-fluorphenyl)-6-isopropyl-(1H)-pyrid-2-on-3,5-dicarbonsäure-3-methyl-5-ethylester

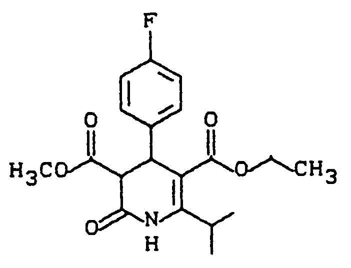

114,3 g (0,48 mol) 1-Carbo methoxy-2-(4-fluorphenyl)-propensäure-methylester, 75,4 g (0,48 mol) 3-Amino-4-methyl-pent-2-en-säureethylester, 1 g Natriummethylat und 5 ml Ethanol wurden 60 h bei 140°C Badtemperatur gerührt und aus Ethanol umkristallisiert.
Schmp.: 124°C
Ausbeute: 115,4 g (66% der Theorie)
- ¹H-NMR (CDCl₃):: δ (ppm) = 1,1 - 1,3 (m, 9H); 3,55 (d, 1H); 3,75 (s, 3H); 4,1 (q, 2H); 4,2 (sept., 1H); 4,65 (d, 1H); 6,9 - 7,2 (m, 4H); 7,7 (s, 1H).

### Beispiel 4

### 4-(4-Fluorphenyl)-6-isopropyl-(1H)-pyrid-2-on-3,5-dicarbonsäure-3-methyl-5-ethylester

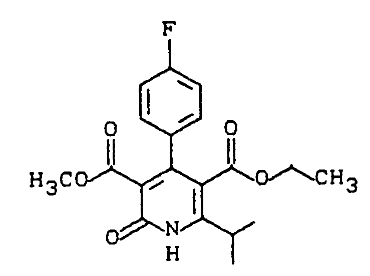

10,8 g (30 mmol) der Verbindung aus Beipiel 3 und 3,9 g (39 mmol) Chromtrioxid wurden in 100 ml Eisessig unter Rückfluß erhitzt, nach 2 h wurden nochmals 2 g (20 mmol) Chromtrioxid zugegeben und über Nacht unter Rückfluß erhitzt. Das Lösemittel wurde abdestilliert, der Rückstand in verdünnter Salzsäure gelöst, mit Ether gewaschen, die vereinigten Etherphasen mit Wasser, wässriger Natriumhydrogencarbonatlösung und Wasser gewaschen, mit Natriumsulfat getrocknet und über Kieselgel 70-230 mesh mit Essigester/Petrolether 1:1 chromatographiert.
Ausbeute: 5,5 g (51% der Theorie)
- ¹H-NMR (CDCl₃):: δ (ppm) = 0,9 (tr, 3H); 1,4 (d, 6H); 3,15 (sept., 1H); 3,6 (s, 3H); 3,9 (q, 2H); 7,0 - 7,3 (m, 4H); 12,2 (s, 1H).

### Beispiel 5

### 4-(4-Fluorphenyl)-6-isopropyl-1-methyl-pyrid-2-on-3,5-dicarbonsäure-3-methyl-5-ethylester

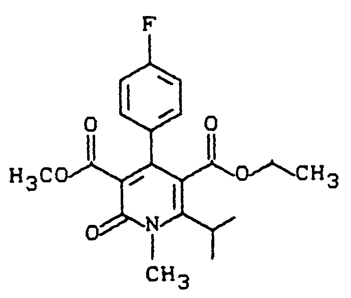

11,3 g (31 mmol) der Verbindung aus Beispiel 4, 1,2 g (50 mmol) Natriumhydrid und 4 ml (62 mmol) Methyliodid werden in 50 ml Dimethylformamid 2 h bei 80°C erhitzt, die Mischung wird bei Raumtemperatur auf 500 ml Wasser gegossen und dreimal mit 150 ml Ether extrahiert. Die vereinigten organischen Phasen werden mit Wasser gewaschen und mit Natriumsulfat getrocknet. Nach Abdestillieren des Lösemittels im Vakuum erhält man 11,1 g.
Rohausbeute: 95,2% der Theorie
- ¹H-NMR (CDCl₃):: δ (ppm) = 0,95 (tr, 3H); 1,3 (d, 6H); 3,15 (sept., 1H); 3,6 (s, 3H); 4,0 (q, 2H); 4,05 (s, 3H); 7,0 - 7,3 (m, 4H).

### Beispiel 6

### 4-(4-Fluorphenyl)-3-hydroxymethyl-6-isopropyl-1-methyl-pyrid-2-on-5-carbonsäureethylester

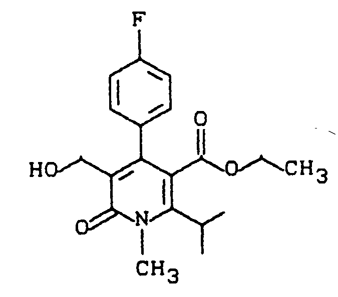

1,48 g (3,95 mmol) der Verbindung aus Beispiel 5 werden in 30 ml Toluol gelöst, unter Stickstoff auf -78°C gekühlt und bei dieser Temperatur 6,6 ml (10 mmol) einer 1,5 molaren Lösung von Diisobutylaluminiumhydrid in Toluol zugetropft. Man entfernt das Kühlbad und rührt 2 h bei Raumtemperatur nach. Nach Hydrolyse mit 20%iger wässriger Kalium-Natriumtartratlösung wird die organische Phase abgetrennt, die wässrige Phase dreimal mit Toluol gewaschen, die vereinigten organischen Phasen mit gesättigter Natriumchloridlösung gewaschen und mit Natriumsulfat getrocknet.
Nach Abdestillieren des Lösemittels im Vakuum erhält man 1,52 g Öl, das über Kieselgel (Essigester/Petrolether 1:5) chromatographiert wird.
Ausbeute: 520 mg (38% der Theorie) und 310 mg (21%) Edukt.
- ¹H-NMR (CDCl₃):: δ (ppm) = 0,95 (tr, 3H); 1,3 (d, 6H); 2,3 (tr, 1H); 3,1 (sept., 1H); 3,95 (q, 2H); 4,05 (s, 3H); 4,4 (d, 2H); 7,0 - 7,3 (m, 4H).

### Beispiel 7

### 4-(4-Fluorphenyl)-6-isopropyl-3-methoxymethyl-1-methyl-pyrid-2-on-5-carbonsäureethylester

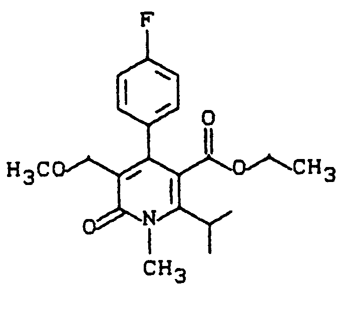

520 mg (1,5 mmol) der Verbindung aus Beispiel 6 werden mit 42 mg (1,75 mmol) Natriumhydrid und 0,3 ml (4,5 mmol) Methyliodid in 4 ml Dimethylformamid 4 h bei Raumtemperatur gerührt. Das Reaktionsgemisch wird auf Eiswasser gegossen, dreimal mit Ether gewaschen, die vereinigten Etherphasen mit Wasser und gesättigter Natriumchloridlösung gewaschen und mit Natriumsulfat getrocknet. Nach Entfernen des Lösemittels am Rotationsverdampfer erhält man 520 mg Öl.
Ausbeute: 100% der Theorie
- ¹H-NMR (CDCl₃):: δ (ppm) = 0,95 (tr, 3H); 1,3 (d, 6H); 3,1 (sept., 1H)); 3,25 (s, 3H); 3,95 (q, 2H); 4,05 (s, 3H); 4,1 (s, 2H); 7,0 - 7,3 (m, 4H).

### Beispiel 8

### 4-(4-Fluorphenyl)-5-hydroxymethyl-6-isopropyl-3-methoxymethyl-1-methyl-pyrid-2-on

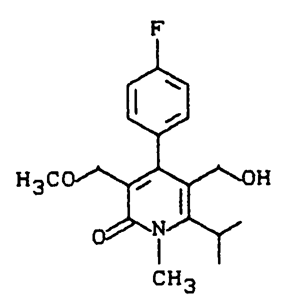

1,19 g (3,5 mmol) der Verbindung aus Beispiel 7 wurden analog Beispiel 6 mit 5,2 ml (7,7 mmol) einer 1,5 molaren Lösung von Diisobutylaluminiumhydrid in Toluol reduziert. Nach Chromatographie über Kieselgel (Essigester/Petrolether 1:5) erhält man 730 mg Feststoff.
Ausbeute: 66% der Theorie
- ¹H-NMR (CDCl₃):: δ (ppm) = 1,2 (tr, 1H); 1,3 (d, 6H); 3,2 (s, 3H); 3,4 (sept., 1H); 4,05 (2s, 5H); 4,35 (d, 2H); 7,1 - 7,3 (m, 4H).

### Beispiel 9

### 4-(4-Fluorphenyl)-6-isopropyl-3-methoxymethyl-1-methyl-pyrid-2-on-5-carbaldehyd

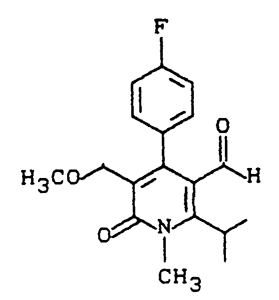

Zu einer Lösung von 0,7 g (2,2 mmol) der Verbindung aus Beispiel 8 in 120 ml Methylenchlorid gibt man 568 mg (2,64 mmol) Pyridiniumchlorochromat, rührt über Nacht bei Raumtemperatur, saugt über Kieselgur ab, wäscht mit 200 ml Methylenchlorid nach, saugt über Kieselgel ab, wäscht mit 200 ml Methylenchlorid nach, trocknet mit Natriumsulfat und erhält nach Entfernen des Lösemittels am Rotationsverdampfer 670 mg Öl.
Ausbeute: 96% der Theorie
- ¹H-NMR (CDCl₃):: δ (ppm) = 1,3 (d, 6H); 3,25 (s, 3H); 4,0 (sept., 1H); 4,08 (s, 2H); 4,10 (s, 3H); 7,1 - 7,3 (m, 4H); 9,7 (s, 1H).

### Beispiel 10

### (E)-3-[4-(4-Fluorphenyl)-6-isopropyl-3-methoxymethyl-1-methyl-pyrid-2-on-5-yl]-prop-2-enal

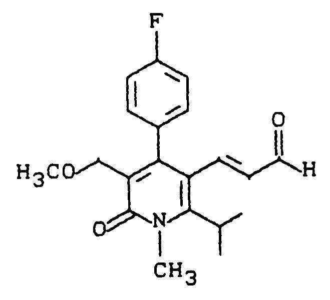

Unter Stickstoff tropft man zu einer Suspension von 59 mg (2,5 mmol) Natriumhydrid in 6 ml trockenem Tetrahydrofuran bei -5°C 804 mg (3,1 mmol) Diethyl-2-(cyclohexylamino)-vinylphosphonat gelöst in 6 ml trockenem Tetrahydrofuran. Nach 30 min. werden bei derselben Temperatur 0,65 g (2,05 mmol) der Verbindung aus Beispiel 9 in 15 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Natriumsulfat getrocknet. Nach Einengen im Vakuum wird der Rückstand in 5 ml Toluol aufgenommen, mit einer Lösung von 0,9 g (7 mmol) Oxalsäure-Dihydrat in 12 ml Wasser versetzt und 90 min zum Rückfluß erhitzt. Nach Abkühlen auf Raumtemperatur werden die Phasen getrennt, die organische Phase mit gesättigter Natriumchlorid-Lösung gewaschen, über Natriumsulfat getrocknet und im Vakuum eingeengt. Der Rückstand wird in Methylenchlorid gelöst und über Kieselgel filtriert.
Ausbeute: 560 mg Feststoff (79,6% der Theorie)
- ¹H-NMR (CDCl₃):: δ (ppm) = 1,3 (d, 6H); 3,25 (s, 3H); 3,35 (sept., 1H); 4,05 (s, 5H); 5,9 (dd, 1H); 7,05 - 7,3 (m, 5H); 9,35 (d, 1H).

### Beispiel 11

### Methyl-(E)-7-[4-(4-fluorphenyl)-6-isopropyl-3-methoxymethyl-1-methyl-pyrid-2-on-5-yl]-5-hydroxy-3-oxo-hept-6-enoat

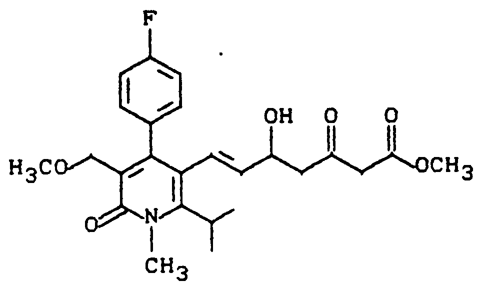

Unter Stickstoff tropft man zu einer Suspension von 80 mg (3,4 mmol) Natriumhydrid in 3 ml trockenem Tetrahydrofuran bei -5°C 0,35 ml (3,3 mmol) Acetessigsäuremethylester. Nach 15 min werden bei derselben Temperatur 2,3 ml (3,3 mmol) 15%iges Butyllithium in n-Hexan und 3,3 ml (3,3 mmol) einer 1 molaren Zinkchlorid-Lösung in Ether zugetropft und 15 min nachgerührt. Anschließend werden 530 mg (1,5 mmol) der Verbindung aus Beispiel 10 gelöst in 8 ml trockenem Tetrahydrofuran zugetropft und 30 min bei -5°C nachgerührt. Die Reaktionslösung wird vorsichtig mit 100 ml gesättigter wässriger Ammoniumchloridlösung verdünnt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Natriumsulfat getrocknet und im Vakuum eingeengt.
Rohausbeute: 760 mg (100% der Theorie)
- ¹H-NMR (CDCl₃):: δ (ppm) = 1,25 (m, 6H); 2,45 (m, 2H), 3,2 (m, 4H); 3,4 (s, 2H); 3,75 (s, 3H); 4,0 (s, 3H); 4,05 (s, 2H); 4,45 (m, 1H); 5,2 (dd, 1H); 6,3 (d, 1H); 7,0 - 7,2 (m, 4H).

### Beispiel 12

### Methyl-erythro-(E)-7-[4-(4-fluorphenyl)-6-isopropyl-3-methoxymethyl-1-methyl-pyrid-2-on-5-yl]-3,5-dihydroxyhept-6-enoat

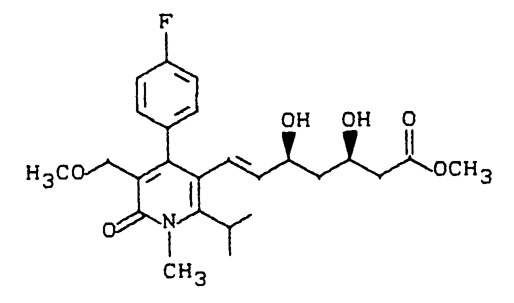

Zu einer Lösung von 730 mg (1,6 mmol) der Verbindung aus Beispiel 11 in 13 ml trockenem Tetrahydrofuran gibt man bei Raumtemperatur 1,9 ml (1,9 mmol) 1 M Triethylboran-Lösung in Tetrahydrofuran, leitet während 5 min Luft durch die Lösung und kühlt auf -30°C Innentemperatur ab. Es werden 72 mg (1,9 mmol) Natriumborhydrid und langsam 1,3 ml Methanol zugegeben, 30 min bei -30°C gerührt und dann mit einem Gemisch von 5 ml 30%igem Wasserstoffperoxid und 11 ml Wasser versetzt. Die Temperatur läßt man dabei bis 0°C ansteigen und rührt noch 30 min nach. Die Mischung wird dreimal mit je 70 ml Essigester extrahiert, die vereinigten organischen Phasen je einmal mit 10%iger Kaliumjodidlösung , 10%iger Natriumthiosulfatlösung, gesättigter Natriumhydrogencarbonat-Lösung und gesättigter Natriumchlorid-Lösung gewaschen, über Natriumsulfat getrocknet und im Vakuum eingeengt. Der Rückstand wird an einer Säule (100 g Kieselgel 230 - 400 mesh, Essigester/Petrolether 1:2) chromatographiert.
Ausbeute: 350 mg Öl (47,6% der Theorie)
- ¹H-NMR (CDCl₃):: δ (ppm) = 1,2 5 (m, 6H); 1,45 (m, 2H); 2,4 (m, 2H); 3,2 (s, 3H); 3,28 (sept., 1H); 3,75 (s, 3H); 4,0 (s, 3H); 4,05 (s, 2H); 4,1 (m, 1H); 4,25 (m, 1H); 5,2 (dd, 1H); 6,25 (d, 1H); 7,0 - 7,2 (m, 4H).

### Beispiel 13

### 4-(4-Fluorphenyl)-3-hydroxymethyl-6-isopropyl-(1H)-pyrid-2-on-5-carbonsäureethylester

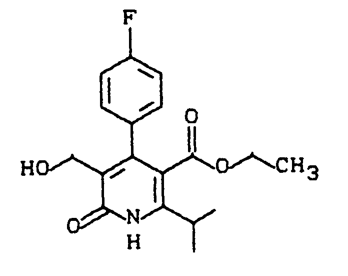

7,02 g (19,45 mmol) der Verbindung aus Beispiel 4 wurden mit 1,17 g (29,2 mmol) Lithiumaluminiumhydrid in 100 ml Tetrahydrofuran 2 h unter Rückfluß erhitzt, mit 20%iger wässriger Kalium-Natriumtartratlösung unter Eiskühlung hydrolysiert und mit Ether gewaschen. Die vereinigten Etherphasen werden mit Wasser gewaschen, mit Natriumsulfat getrocknet und nach Entfernen des Lösemittels durch Chromatographie über Kieselgel (Methylenchlorid/Methanol 20:1) gereinigt.
Ausbeute: 1,09 g (16,8% der Theorie)
- ¹H-NMR (CDCl₃):: δ (ppm) = 0,9 (tr, 3H); 1,4 (d, 6H); 3,15 (sept., 1H); 3,9 (q, 2H); 4,05 (tr, 1H); 4,4 (d, 2H); 7,05 - 7,3 (m, 4H); 12,4 (s, 1H).

### Beispiel 14

### 1,6-Diisopropyl-4-(4-fluorphenyl)-3-hydroxymethyl-pyrid-2-on-5-carbonsäureethylester

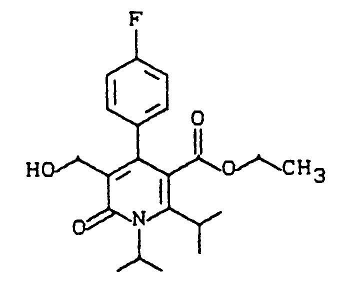

1,6 g (4,8 mmol) der Verbindung aus Beispiel 13, 1,7 ml (17,3 mmol) 2-Jodpropan und 2,3 g Kaliumcarbonat werden 5 h in 30 ml Aceton unter Rückfluß erhitzt, nach Filtration und Entfernen des Lösemittels wird der Rückstand in Methylenchlorid aufgenommen, mit Wasser gewaschen, mit Natriumsulfat getrocknet und über Kieselgel (Methylenchlorid/Methanol 40:1) chromatographiert.
Ausbeute: 1,14 g (63% der Theorie)
- ¹H-NMR (CDCl₃):: δ (ppm) = 0,95 (tr, 3H); 1,3 (d, 6H); 1,45 (d, 6H); 2,5 (tr, 1H); 3,1 (sept., 1H); 3,95 (q, 4H); 4,35 (d, 2H); 5,5 (sept., 1H); 7,0 - 7,3 (m, 4H).

### Beispiel 15

### 1,6-Diisopropyl-4-(4-fluorphenyl)-3-methoxymethyl-pyrid-2-on-5-carbonsäureethylester

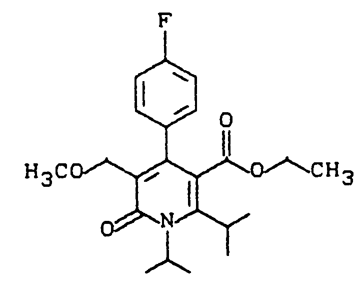

Analog Beispiel 7 erhält man ausgehend von 1,1 g (2,93 mmol) der Verbindung aus Beispiel 14, 1,1 ml (17,6 mmol) Methyljodid und 155 mg (6,45 mmol) Natriumhydrid, 1,04 g Öl.
Rohausbeute: 91% der Theorie
- ¹H-NMR (CDCl₃):: δ (ppm) = 0,95 (tr, 3H); 1,25 (d, 6H); 1,4 (d, 6H); 3,1 (sept., 1H); 3,25 (s, 3H); 3,95 (q, 2H); 4,1 (s, 2H); 5,45 (sept., 1H); 7,0 - 7,4 (m, 4H).

### Beispiel 16

### 1,6-Diisopropyl-4-(4-fluorphenyl)-5-hydroxymethyl-3-methoxymethyl-pyrid-2-on

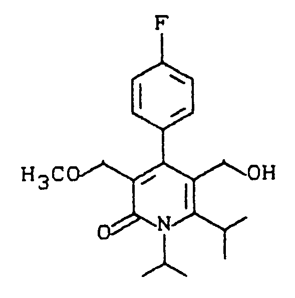

Analog Beispiel 8 erhält man ausgehend von 1,02 g (2,57 mmol) der Verbindung aus Beispiel 15 680 mg der Titelverbindung.
Ausbeute: 76,2% der Theorie
- ¹H-NMR (CDCl₃):: δ (ppm) = 1,15 (tr, 1H); 1,3 (d, 6H); 1,4 (d, 6H); 3,2 (s, 3H); 3,4 (sept., 1H); 4,05 (s, 2H); 4,35 (d, 2H); 5,4 (sept., 1H); 7,05 - 7,3 (m, 4H).

### Beispiel 17

### 1,6-Diisopropyl-4-(4-fluorphenyl)-3-methoxymethyl-pyrid-2-on-3-carbaldehyd

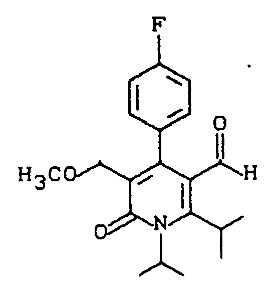

Analog Beispiel 9 erhält man ausgehend von 680 mg (1,96 mmol) der Verbindung des Beispiels 16 620 mg der Titelverbindung.
Ausbeute: 91,6% der Theorie
- ¹H-NMR (CDCl₃):: δ (ppm) = 1,25 (d, 6H); 1,45 (d, 6H); 3,25 (s, 3H); 4,0 (sept., 1H); 4,05 (s, 2H); 5,5 (sept., 1H); 7,1 - 7,3 (m, 4H); 9,65 (s, 1H).

### Beispiel 18

### (E)-3-[1,6-Diisopropyl-4-(4-fluorphenyl)-3-methoxymethyl-pyrid-2-on-5-yl]-prop-2-enal

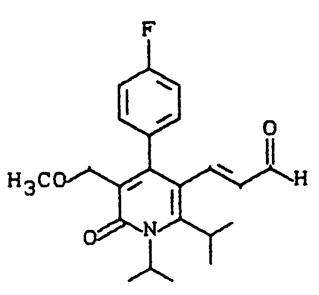

Analog Beispiel 10 erhält man ausgehend von 620 mg (1,8 mmol) der Verbindung des Beispiels 17 550 mg der Titelverbindung.
Ausbeute: 82,5% der Theorie
- ¹H-NMR (CDCl₃):: δ (ppm) = 1,25 (d, 6H), 1,40 (d, 6H); 3,2 (s, 3H); 3,30 (m, 1H); 4,05 (s, 2H); 5,45 (m, 1H); 5,85 (dd, 1H); 7,0 - 7,2 (m, 5H).

### Beispiel 19

### Methyl-(E)-7-[1,6-diisopropyl-4-(4-fluorphenyl)-3-methoxymethyl-pyrid-2-on-5-yl]-5-hydroxy-3-oxo-hept-6-enoat

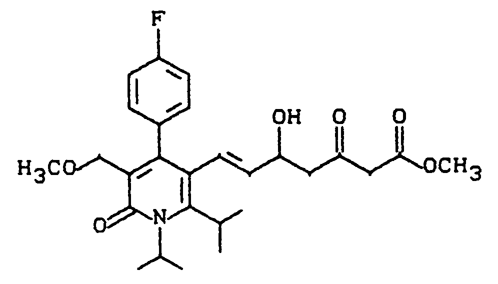

Analog Beispiel 11 erhält man ausgehend von 520 mg (1,4 mmol) der Verbindung aus Beispiel 18 1,11 g Rohprodukt.
Rohausbeute: 100% der Theorie
- ¹H-NMR (CDCl₃):: δ (ppm) = 1,15 - 1,45 (m, 12H); 2,4 (m, 2H); 3,25 (m, 4H); 3,45 (s, 2H); 3,75 (s, 3H); 4,05 (s, 2H); 4,5 (m, 1H); 5,2 (dd, 1H); 5,4 (m, 1H); 6,3 (d, 1H); 7,0 - 7,2 (m, 4H).

### Beispiel 20

### Methyl-erythro-(E)-7-[1,6-diisopropyl-4-(4-fluorphenyl)-3-methoxymethyl-pyrid-2-on-5-yl]-3,5-dihydroxy-hept-6-enoat

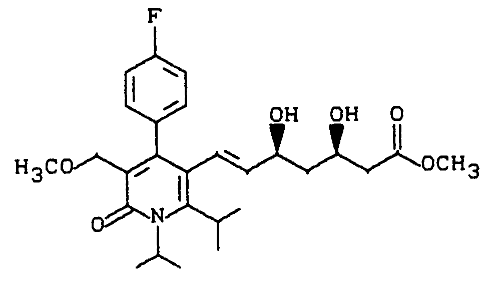

Analog Beispiel 12 erhält man ausgehend von 1,05 g (2,16 mmol) der Verbindung des Beispiels 19 240 mg Öl.
Ausbeute: 22,7% der Theorie
- ¹H-NMR (CDCl₃):: δ (ppm) = 1,1 - 1,5 (m, 14H); 2,40 (m, 2H); 3,25 (m, 4H); 3,75 (s, 3H); 4,05 (m, 3H); 4,30 (m, 1H); 5,15 (dd, 1H); 5,40 (m, 1H); 6,25 (d, 1H); 6,95 - 7,2 (m, 4H).

### Beispiel 21

### 3,5-Dihydroxymethyl-4-(4-fluorphenyl)-6-isopropyl-1-methyl-pyrid-2-on

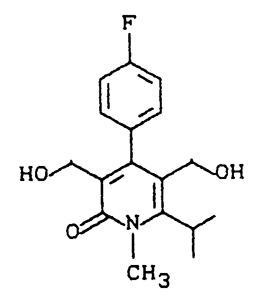

Ausgehend von 3,0 g (8 mmol) der Verbindung des Beispiels 5 und 26,6 ml (40 mmol) einer 1,5 m Lösung von Diisobutylaluminiumhydrid in Toluol erhält man analog Beispiel 6 2,64 g der Titelverbindung.
Rohausbeute: 100% der Theorie
- ¹H-NMR (CDCl₃):: δ (ppm) = 1,20 (tr, 1H); 1,35 (d, 6H); 2,40 (tr, 1H); 3,45 (m, 1H); 4,05 (s, 3H); 4,30 (d, 2H); 4,35 (d, 1H); 7,1 - 7,3 (m, 4H).

### Beispiel 22

### 4-(4-Fluorphenyl)-6-isopropyl-1-methyl-pyrid-2-on-3,5-dicarbaldehyd

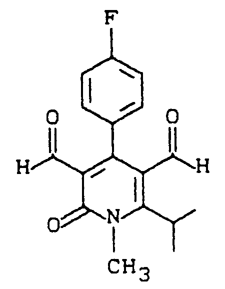

Analog Beispiel 9 erhält man ausgehend von 2,60 g (8,5 mmol) der Verbindung aus Beispiel 21 2,13 g der Titelverbindung.
Ausbeute: 83,3% der Theorie
- ¹H-NMR (CDCl₃):: δ (ppm) = 1,35 (d, 6H); 4,0 (m, 1H); 4,2 (s, 3H); 7,15 - 7,3 (m, 4H); 9,65 (s, 1H); 9,95 (s, 1H).

### Beispiel 23

### (E,E)-3,3-[4-(4-Fluorphenyl)-6-isopropyl-1-methyl-pyrid-2-on-3,5-diyl]-diprop-2-enal

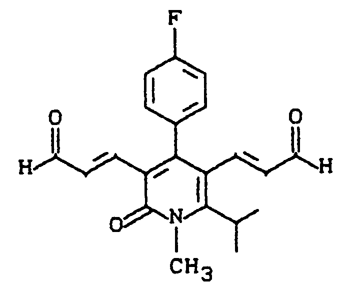

Analog Beispiel 10 erhält man ausgehend von 2,13 g (7,1 mmol) der Verbindung des Beispiels 22 2,70 g Rohprodukt.
Ausbeute: 100% der Theorie
- ¹H-NMR (CDCl₃):: δ (ppm) = 1,30 (d, 6H); 3,30 (m, 1H); 4,15 (s, 3H); 5,95 (dd, 1H); 7,0 - 7,25 (m, 5H); 9,3 - 9,4 (m, 2H).

### Beispiel 24

### 3,5-Di-[methyl-(E)-hydroxy-3-oxo-hept-6-enoat-7-yl]-4-(4-fluorphenyl)-6-isopropyl-1-methyl-pyrid-2-on

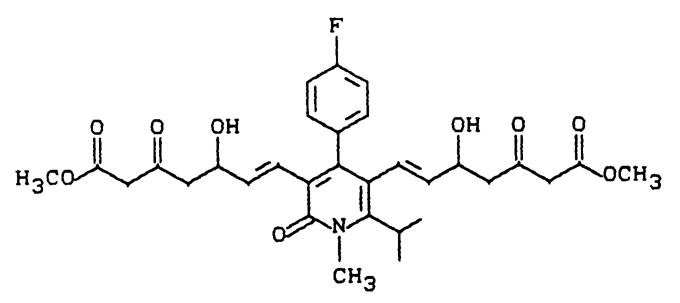

Analog Beispiel 11 erhält man ausgehend von 0,31 g (0,88 mmol) der Verbindung des Beispiels 23 1,04 g Rohprodukt.
Ausbeute: 100% der Theorie
- ¹H-NMR (CDCl₃):: δ (ppm) = 1,1 - 1,4 (m, 6H); 2,3 - 2,7 (m, 4H); 3,2 (m, 1H); 3,45 (m, 4H); 3,75 (m, 6H); 4,05 (s, 3H); 4,5 (m, 2H); 5,2 (m, 2H); 6,2 (m, 2H); 6,8 - 7,2 (m, 4H).

### Beispiel 25

### 3,5-Di-[methyl-erythro-(E)-3,5-dihydroxy-hept-6-enoat-7-yl]-4-(4-fluorphenyl)-6-isopropyl-1-methyl-pyrid-2-on

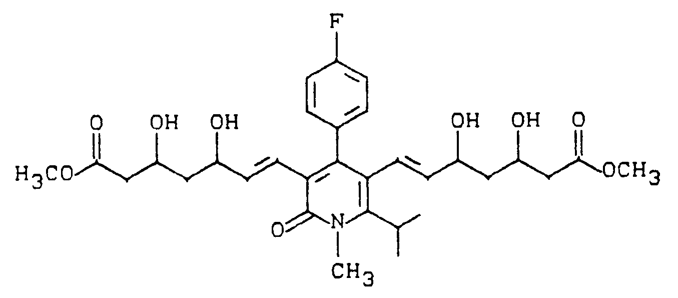

Analog Beispiel 12 erhält man ausgehend von 1,04 g (0,88 mmol) der Verbindung des Beispiels 24 nach Chromatographie über Kieselgel (Essigester/Petrolether 1:1) 74 mg.
Ausbeute: 14,3% der Theorie
- ¹H-NMR (CDCl₃):: δ (ppm) = 1,25 (m, 6H); 1,6 (m, 4H); 2,45 (m, 4H); 3,30 (m, 1H); 3,75 (2s, 6H); 4,05 (s, 3H); 4,15 (m, 2H); 4,30 (m, 2H); 5,25 (dd, 2H); 6,2 (m, 2H); 6,95 - 7,15 (m, 4H).

### Beispiel 26

### 3-Benzyloxymethyl-4-(4-fluorphenyl)-6-isopropyl-1-methyl-pyrid-2-on-5-carbonsäureethylester

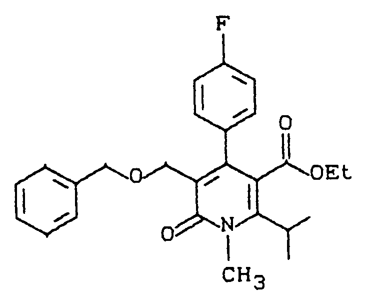

Analog Beispiel 7 erhält man ausgehend von 630 mg (1,9 mmol) der Verbindung aus Beispiel 6 und 720 mg Benzylbromid die Titelverbindung.
Ausbeute: 92,2 % der Theorie
- ¹H-NMR (CDCl₃):: δ (ppm) = 0,9 (t, 3H); 1,3 (d, 6H); 3,05 (sept., 1H); 3,95 (q, 2H); 4,03 (s, 3H); 4,2 (s, 2H); 4,4 (s, 2H); 7,0-7,4 (m, 9H);

### Beispiel 27

### 3-Benzyloxymethyl-4-(4-fluorphenyl)-5-hydroxymethyl-6-isopropyl-1-methyl-pyrid-2-on

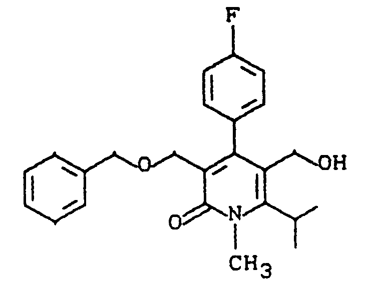

Analog der Vorschrift für Beispiel 8 erhält man ausgehend von 700 mg (1,7 mmol) der Verbindung aus Beispiel 26 520 mg der Titelverbindung.
Ausbeute: 77,4 % der Theorie
- ¹H-NMR (CDCl₃):: δ (ppm) = 1,32 (d, 6H); 3,4 (sept., 1H); 4,02 (s, 3H); 4,15 (s, 2H); 4,3 (s, 2H); 4,38 (s, 2H); 7,0-7,4 (m, 9H).

### Beispiel 28

### 3-Benzyloxymethyl-4-(4-fluorphenyl)-6-isopropyl-1-methyl-pyrid-2-on-5-carbaldehyd

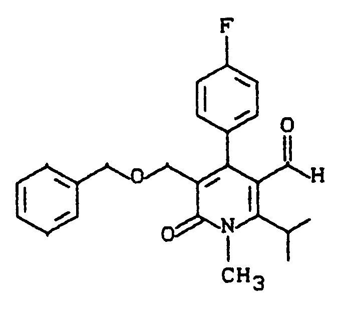

Analog Beispiel 9 erhält man ausgehend von 500 mg (1,3 mmol) der Verbindung des Beispiels 27 400 mg der Titelverbindung.
Ausbeute: 78,3 % der Theorie
- ¹H-NMR (CDCl₃):: δ (ppm) = 1,25 (d, 6H); 4,0 (sept., 1H); 4,08 (s, 3H); 4,15 (s, 2H); 4,4 (s, 2H); 7,0-7,4 (m, 9H); 9,65 (s, 1H).

### Beispiel 29

### (E)-3-[3-Benzyloxymethyl-4-(4-fluorphenyl)-6-isopropyl-1-methyl-pyrid-2-on-5-yl]-prop-2-enal

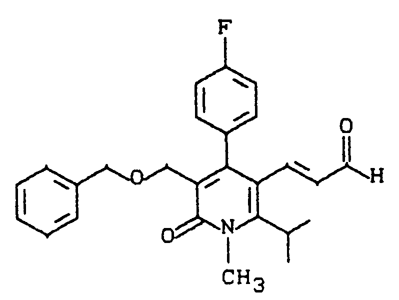

Analog der Vorschrift für Beispiel 10 erhält man ausgehend von 380 mg (0,97 mmol) der Verbindung aus Beispiel 28 400 mg der Titelverbindung.
Ausbeute: 78,3 % der Theorie
- ¹H-NMR (CDCl₃):: δ (ppm) = 1,28 (d, 6H); 3,32 (sept.,1H); 4,03 (s, 3H); 4,15 (s, 2H); 4,38 (s, 2H); 5,88 (dd, 1H); 7,0-7,4 (m, 10H); 9,35 (d,1H).

### Beispiel 30

### Methyl-(E)-7-[3-Benzyloxymethyl-4-(4-fluorphenyl)-6-isopropyl-1-methyl-pyrid-2-on-5-yl]-5-hydroxy-3-oxo-hept-6-enoat

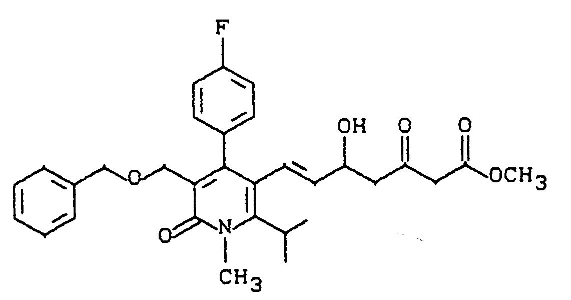

Analog Beispiel 11 erhält man ausgehend von 400 mg (0,76 mmol) der Verbindung des Beispiels 29 70 mg der Titelverbindung.
Ausbeute: 20,9 % der Theorie
- ¹H-NMR (CDCl₃):: δ (ppm) = 1,25 (m, 6H); 2,45 (m, 2H); 3,22 (m, 1H); 3,41 (s, 2H); 3,72 (s, 3H); 4,0 (s, 3H); 4,15 (s, 2H); 4,4 (s, 2H); 4,48 (m, 1H); 5,18 (dd, 1H); 6,28 (d, 1H); 7,0-7,4 (m, 9H).

### Beispiel 31

### Methyl-erythro-(E)-7-[3-Benzyloxymethyl-4-(4-fluorphenyl)-6-isopropyl-1-methyl-pyrid-2-on-5-yl]-3,5-dihydroxy-hept-6-enoat

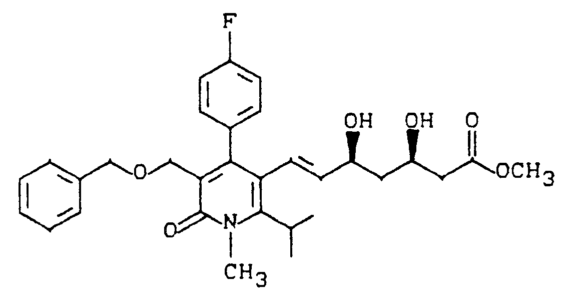

Analog Beispiel 12 erhält man ausgehend von 70 mg (0,13 mmol) der Verbindung des Beispiels 30 42 mg der Titelverbindung als Öl.
Ausbeute: 60,2 % der Theorie
- ¹H-NMR (CDCl₃):: δ (ppm) = 1,1-1,5 (m, 8H); 2,4 (m, 2H); 3,25 (sept., 1H); 3,72 (s, 3H); 4,02 (s, 3H); 4,08 (m, 1H); 4,15 (s, 2H); 4,3 (m, 1H); 4,42 (s, 2H); 5,2 (dd, 1H); 6,26 (d, 1H); 7,0-7,4 (m, 9H).

### Beispiel 32

### 3-(tert.-Butyldimethylsilyloxymethyl-4-(4-fluorphenyl)-6-isopropyl-1-methyl-pyrid-2-on-5-carbonsäureethylester

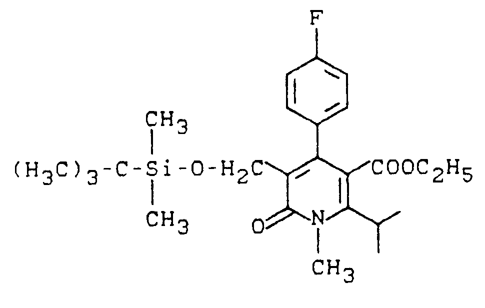

Zu einer Lösung von 600 mg (1,8 mmol) der Verbindung aus Beispiel 6 in 20 ml Dimethylformamid gibt man bei Raumtemperatur 304 mg (2 mmol) tert.-Butyldimethylsilylchlorid, 262 mg (4 mmol) Imidazol und 0,05 g 4-Dimethylaminopyridin. Es wird über Nacht bei Raumtemperatur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Magnesiumsulfat getrocknet und im Vakuum eingeengt. Der Rückstand wird an einer Säule (150 g Kieselgel, 70-230 mesh, ⌀ 4 cm, mit Essigester/Petrolether 1:9) chromatographiert.
Ausbeute: 700 mg (87 % der Theorie)
- ¹H-NMR (CDCl₃):: δ = 0,0 (s, 6H); 0,85 (s, 9H); 0,95 (t, 3H); 1,3 (d, 6H); 3,1 (m, 1H); 3,95 (q, 2H); 4,0 (s, 2H); 4,35 (s, 3H); 7,05 (m, 2H); 7,35 (m, 2H) ppm.

### Beispiel 33

### Methyl-erythro-(E)-7-[3-tert.-butyldimethylsilyloxymethyl-4-(4-fluorphenyl)-6-isopropyl-1-methyl-pyrid-2-on-5-yl]-5-dihydroxy-hept-6-enoat

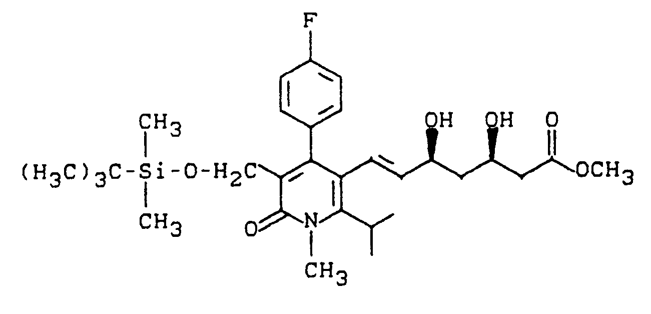

Ausgehend von Beispiel 32 wurde analog zu den Vorschriften der Beispiele 8-12 die Titelverbindung hergestellt.
- ¹H-NMR (CDCl₃):: δ = 0,0 (s, 6H); 0,9 (s, 9H); 1,25 (m, 6H); 1,5 (m, 2H); 2,45 (m, 2H); 2,8 (m, 1H); 3,3 (m, 1H); 3,6 (m, 1H); 3,75 (s, 3H); 4,0 (s, 3H); 4,1 (m, 1H); 4,3 (m, 3H); 5,2 (dd, 1H); 6,3 (d, 1H); 7,0-7,3 (m, 4H) ppm.

### Beispiel 34

### Methyl-erythro-(E)-7-[4-(4-fluorphenyl-3-hydroxymethyl-6-isopropyl-1-methyl-pyrid-2-on-5-yl]-3,5-dihydroxy-hept-6-enoat

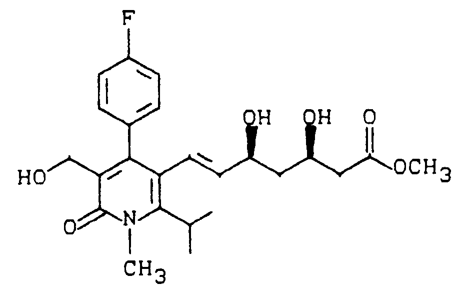

100 mg (0,18 mmol) der Verbindung aus Beispiel 33 werden in einer Lösung von 1 ml 1 N-Salzsäure und 9 ml Methanol über Nacht bei Raumtemperatur gerührt. Nach dem Einengen wird mit Methylchlorid aufgenommen, mit gesättigter Natriumhydrogencarbonatlösung gewaschen, getrocknet und über Kieselgel filtriert (Essigester/Petrolether 1:1).
Ausbeute: 46 mg (57 % der Theorie)
- ¹H-NMR (CDCl₃):: δ = 1,2 (m, 6H); 1,4 (m, 2H); 2,4 (m, 2H); 3,4 (m, 1H); 3,3 (m, 1H); 3,55 (m, 1H); 3,7 (s, 3H); 4,05 (s, 3H); 4,1 (m, 1H); 4,35 (m, 3H); 5,2 (dd, 1H); 6,3 (d, 1H); 7,0-7,2 (m, 4H) ppm.

### Beispiel 35

### 1-Carbomethoxy-2-phenyl-propensäure-methylester

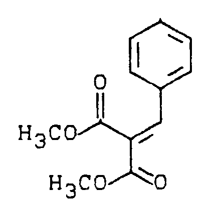

Analog Beispiel 2 wurde aus Benzaldehyd und Malonsäuredimethylester die Titelverbindung erhalten.
Ausbeute: 97,3 % der Theorie
Sdp.: 131°C/12 mm
- ¹H-NMR (CDCl₃):: δ = 3,75 (s, 6H); 7,4 (m, 5H); 7,8 (s, 1H) ppm.

### Beispiel 36

### Methyl-erythro-(E)-7-[6-isopropyl-3-methoxymethyl-1-methyl-4-phenyl-pyrid-2-on-5-yl]-3,5-dihydroxy-hept-6-enoat

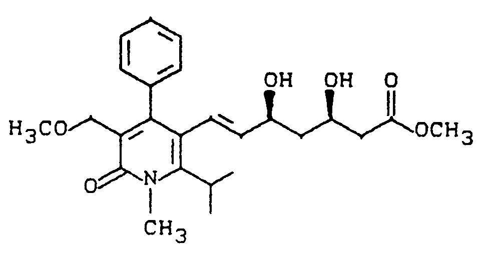

Ausgehend von Beispiel 35 wurde in Analogie zu den Vorschriften der Beispiele 3-12 die Titelverbindung erhalten.
- ¹H-NMR (CDCl₃):: δ = 1,2 (m, 6H); 1,4 (m, 2H); 2,4 (m, 2H); 2,6 (s, 1H); 3,2 (s, 3H); 3,25 (m, 1H); 3,5 (m, 1H); 3,7 (s, 3H); 4,0 (s, 3H); 4,1 (s, 2H); 4,05 (m, 1H); 4,25 (m, 1H); 5,2 (dd, 1H); 6,3 (d, 1H); 7,1-7,5 (m, 5H) pm.

### Beispiel 37

### 3-Amino-3-cyclopropyl-prop-2-en-säure-ethylester

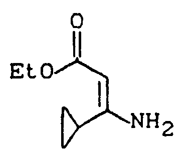

Analog Beispiel 1 wurde die Titelverbindung aus Cyclopropyl-carbonylessigsäureethylester erhalten.
Sdp.: 63°C/0,3 mbar
Ausbeute: 24 % der Theorie

### Beispiel 38

### Methyl-erythro-(E)-7-[6-cyclopropyl-4-(4-fluorphenyl)-3-methoxymethyl-1-methyl-pyrid-2-on-5-yl]-3,5-dihydroxy-hept-6-enoat

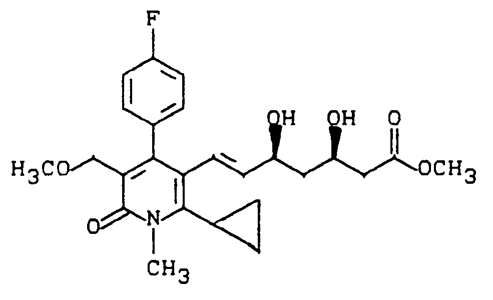

Ausgehend von Beispiel 37 wurde die Titelverbindung in Analogie zu den Vorschriften der Beispiele 3-12 hergestellt.
- ¹H-NMR (CDCl₃):: δ = 0,95 (m, 2H); 1,15 (m, 2H); 1,35 (m, 2H); 2,25 (m, 1H); 2,45 (m, 2H); 2,75 (s, 1H); 3,2 (s, 3H); 3,5 (s, 1H); 3,7 (s, 3H); 3,95 (s, 3H); 4,05 (s, 2H); 4,1 (m, 1H); 4,3 (m, 1H); 5,5 (dd, 1H); 6,3 (d, 1H); 7,0-7,2 (m, 4H) ppm.

In Analogie zur Vorschrift für Beispiel 5 wurden durch Alkylierung mit Ethyliodid, Benzylbromid und 4-Methoxybenzylchlorid die entsprechenden N-Alkylderivate hergestellt, die wiederum in Analogie zu den Vorschriften der Beispiele 6-12 zu den im folgenden aufgeführten Beispielen 39, 40 und 41 umgesetzt wurden.

### Beispiel 39

### Methyl-erythro-(E)-7-[1-ethyl-4-(4-fluorphenyl)-6-isopropyl-3-methoxymethyl-pyrid-2-on-5-yl]-3,5-dihydroxy-hept-6-enoat

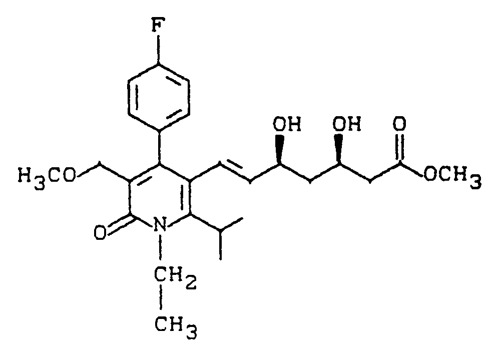

- ¹H-NMR (CDCl₃):: δ = 1,2 (m, 6H); 1,4 (m, 5H); 2,45 (m, 2H); 2,7 (s, 1H); 3,2 (s, 3H); 3,25 (m, 1H); 3,5 (s, 1H); 3,7 (s, 3H); 4,05 (m, 3H); 4,3 (m, 1H); 4,5 (q, 2H); 5,2 (dd, 1H); 6,25 (d, 1H); 7,0-7,2 (m, 4H) ppm.

### Beispiel 40

### Methyl-erythro-(E)-7-[1-benzyl-4-(4-fluorphenyl)-6-isopropyl-3-methoxymethyl-pyrid-2-on-5-yl]-3,5-dihydroxy-hept-6-enoat

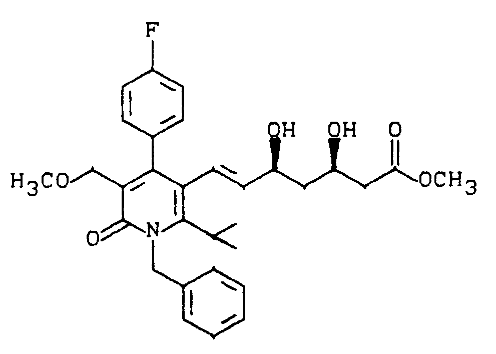

- ¹H-NMR (CDCl₃):: δ = 1,2 (m, 6H); 1,45 (m, 2H); 2,4 (m, 2H); 2,3 (s, 1H); 3,2 (s, 3H); 3,25 (m, 1H); 3,5 (s, 1H); 3,7 (s, 3H); 4,05 (m, 3H); 4,25 (m, 1H); 5,2 (dd, 1H); 5,5 (s, 2H); 6,25 (d, 1H); 7,0-7,5 (m, 9H) ppm.

### Beispiel 41

### Methyl-erythro-(E)-7-[4-(4-fluorphenyl)-6-isopropyl-3-methoxymethyl-1-(4-methoxyphenyl)-pyrid-2-on-5-yl]-3,5-dihydroxy-hept-6-enoat

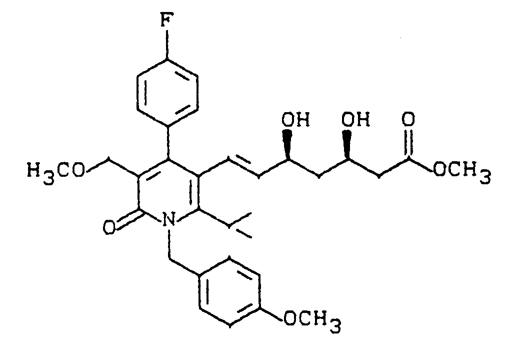

- ¹H-NMR (CDCl₃):: δ = 1,2 (m, 6H); 1,45 (m, 2H); 2,4 (m, 2H); 2,7 (s, 1H); 3,2 (s, 3H); 3,25 (m, 1H); 3,5 (s, 1H); 3,7 (s, 3H); 3,8 (s, 3H); 4,1 (m, 3H); 4,3 (m, 1H); 5,2 (dd, 1H); 5,45 (s, 2H); 6,25 (d, 1H); 6,8-7,5 (m, 8H) ppm.

### Beispiel 42

### 3,4-Dihydro-4-(4-fluorphenyl)-6-isopropyl-(1H)-pyrid-2-on-5-carbonsäure-ethylester

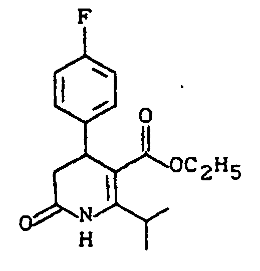

20,0 g (55 mmol) der Verbindung aus Beispiel 3 und 3,3 g Natriumchlorid wurden in 55 ml Dimethylsulfoxid und 2,5 ml Wasser 2,5 h bei 180°C gerührt und nach dem Abkühlen auf Eiswasser gegeben. Der ausgefallene Feststoff wurde abgesaugt und aus Ethanol umkristallisiert.
Schmp.: 119-120°C
Ausbeute: 12,6 g (75 % der Theorie)

### Beispiel 43

### Methyl-erythro-(E)-7-[4-(4-fluorphenyl)-6-isopropyl-1-methyl-pyrid-2-on-5-yl]-3,5-dihydroxy-hept-6-enoat

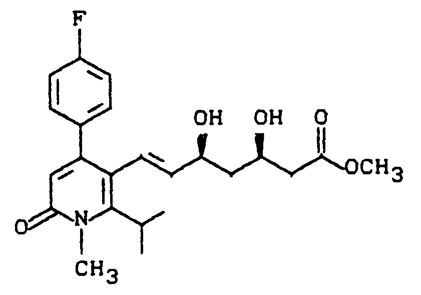

Ausgehend von Beispiel 42 wurde die Titelverbindung analog den Vorschriften der Beispiele 4, 5 und 8-12 erhalten.
- ¹H-NMR (CDCl₃):: δ = 1,2 (d, 6H), 1,5 (m, 2H); 2,45 (m, 2H); 3,0 (s, 1H); 3,3 (m, 1H); 3,6 (s, 1H); 3,7 (s, 3H); 3,95 (s, 3H); 4,1 (m, 1H); 4,4 (m, 1H); 5,25 (dd, 1H); 6,45 (m, 2H); 7,0-7,3 (m, 4H) ppm.

### Anwendungsbeispiel

Die Serum-Cholesterin-senkende Wirkung der erfindungsgemäßen Verbindungen auf die Blutcholesterinwerte von Hunden wurde in mehrwöchigen Fütterungsexperimenten gefunden. Dazu wurde die zu untersuchende Substanz über einen mehrwöchigen Zeitraum einmal täglich in einer Kapsel gesunden Beagle Hunden zusammen mit dem Futter p. o. gegeben. Dem Futter war außerdem während der gesamten Versuchsperiode, d. h. vor, während und nach der Applikationsperiode der zu untersuchenden Substanz Colestyramin (4 g/100 g Futter) als Gallensäuresequestrant beigemischt.

Zweimal wöchentlich wurde den Hunden venöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

## Patentansprüche (Patentansprüche für folgende(n) Vertragsstaat(en): AT, BE, CH, DE, FR, GB, GR, IT, LI, LU, NL, SE)

1. 2-Pyridone und Pyrid-2-thione der allgemeinen Formel (Ia) und (Ib) 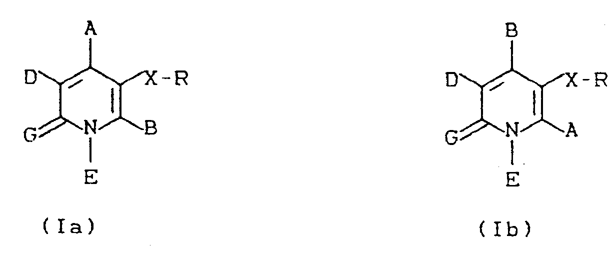 in welcher
A
- für Oxiranyl, Thienyl, Furyl, Pyridyl, Pyrimidyl, Pyrazinyl, Indolyl, Chinolyl, Isochinolyl, Benzothiazolyl oder Benzimidazolyl steht, das gegebenenfalls bis zu 4-fach gleich oder verschieden durch Fluor, Chlor, Brom, Trifluormethyl, Trifluormethylthio, Trifluormethoxy, geradkettiges oder verzweigtes Alkyl, Alkylthio, Alkylsulfonyl, Alkoxy oder Alkoxycarbonyl mit jeweils bis zu 6 Kohlenstoffatomen, Phenyl, Phenylthio, Phenylsulfonyl oder durch eine Gruppe der Formel -NR¹R² substituiert ist,
worin
R¹ und R² gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel -COR³ bedeuten,
worin
R³
- geradkettiges oder verzweigtes Alkyl oder Alkoxy mit bis zu 6 Kohlenstoffatomen oder Phenyl bedeutet,
oder
R¹ und R² gemeinsam mit dem Stickstoffatom einen 5- bis 7-gliedrigen Ring bilden, der durch geradkettiges oder verzweigtes Alkyl mit bis zu 6 Kohlenstoffatomen substituiert sein kann,
- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy, Alkoxy mit bis zu 4 Kohlenstoffatomen, Phenyl oder durch eine Gruppe der Formel -NR¹R² substituiert sein können, oder durch Phenyl, Phenyloxy, Phenylthio, Phenylsulfonyl, Fluor, Chlor, Brom, Nitro, Cyano, Trifluormethyl, Trifluormethoxy, Trifluormethylthio, Benzyloxy oder durch eine Gruppe der Formel -NR¹R² substituiert ist,
B
- für Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, Cyano, Azido, Trifluormethyl, Trifluormethoxy, Trifluormethylthio, Trifluormethylsulfonyl, Alkoxy mit bis zu 8 Kohlenstoffatomen oder durch Phenyl, Phenyloxy, oder Phenylthio, Thienyl, Furyl, Pyridyl, Pyrimidyl oder Chinolyl substituiert ist, welches seinerseits bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein kann, oder
durch eine Gruppe der Formel -NR¹R² oder -COR³ substituiert ist
- für Phenyl steht, das gegebenenfalls bis zu 2-fach gleich oder verschieden durch Fluor, Chlor, Brom, Cyano, Nitro, Trifluormethyl, geradkettiges oder verzweigtes Alkyl, Alkoxy oder Alkoxycarbonyl mit jeweils bis zu 6 Kohlenstoffatomen oder Amino substituiert ist,
D und E gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclobutyl, Cyclopentyl oder Cyclohexyl stehen,
- für geradkettiges oder verzweigtes Alkyl oder Alkenyl mit jeweils bis zu 10 Kohlenstoffatomen oder Imino stehen, die gegebenenfalls durch Fluor, Chlor, Brom, Azido, 2,5-Dioxotetrahydropyrryl, Phenyl, Pyrimidyl, Pyrryl, Pyrrolidinyl, Morpholino oder Morpholino-N-oxid substituiert ist, oder durch eine Gruppe der Formel NR¹R², -OR⁴, -COR⁵ oder
-S(O)ₙ-R⁶ substituiert sind,
worin
R¹ und R² die oben angegebene Bedeutung haben,
R⁴
- Wasserstoff oder
- geradkettiges oder verzweigtes Alkyl mit bis zu 8 Kohlenstoffatomen bedeutet, das gegebenenfalls durch Hydroxy, Trialkylsilyl mit bis zu 8 Kohlenstoffatomen im gesamten Alkylteil, Fluor, Chlor, Brom oder durch Phenyl substituiert ist, welches seinerseits durch Fluor, Chlor, Brom, Cyano, Nitro, Hydroxy, geradkettiges oder verzweigtes Alkyl, Alkoxy oder Alkoxycarbonyl mit jeweils bis zu 6 Kohlenstoffatomen oder Amino substituiert sein kann,
- Trialkylsilyl mit bis zu 8 Kohlenstoffatomen im gesamten Alkylteil, Tetrahydropyranyl oder 2,5-Dioxo-tetrahydropyrryl bedeutet,
- Cyclopropyl, Cyclobutyl, Cyclopentyl, Cyclohexyl oder Phenyl bedeutet, welches seinerseits durch Fluor, Chlor, Brom, Cyano, Nitro oder Amino substituiert sein kann, oder
- eine Gruppe der Formel -COR⁷ bedeutet,
worin
R⁷
- geradkettiges oder verzweigtes Alkyl mit bis zu 6 Kohlenstoffatomen, Phenyl oder eine Gruppe der Formel -NR¹R² bedeutet,
R⁵
- Wasserstoff, geradkettiges oder verzweigtes Alkyl mit bis zu 8 Kohlenstoffatomen bedeutet, das gegebenenfalls durch Hydroxy, Phenyl, Fluor, Chlor, Brom oder Cyano substituiert ist,
- Phenyl, Naphthyl, Pyrryl, Pyrimidyl, Pyridyl, Pyrrolidinyl oder Morpholino bedeutet, welches seinerseits durch Fluor, Chlor, Brom, Amino, Hydroxy, Nitro oder Cyano substituiert sein kann, oder
- eine Gruppe der Formel -NR¹R² oder -OR⁴ bedeutet,
n
- eine Zahl 0 oder 2 bedeutet,
R⁶
- geradkettiges oder verzweigtes Alkyl mit bis zu 8 Kohlenstoffatomen bedeutet, das durch Fluor, Chlor, Brom, Hydroxy, Phenyl oder durch eine Gruppe der Formel -NR¹R² substituiert sein kann,
- Phenyl bedeutet, das durch Fluor, Chlor, Brom, Hydroxy, Cyano, Nitro oder Amino substituiert sein kann, oder
- eine Gruppe der Formel -NR¹R² bedeutet, wenn n für die Zahl 2 steht,
oder
D und E gleich oder verschieden sind und
- für eine Gruppe der Formel -NR¹R², -OR⁴ oder -COR⁵ stehen,
worin
R¹, R², R⁴ und R⁵ die oben angegebene Bedeutung haben,
D oder E gemeinsam mit B einen 5- bis 7-gliedrigen, gesättigten oder ungesättigten Ring bilden, der gegebenenfalls durch geradkettiges oder verzweigtes Alkyl mit bis zu 6 Kohlenstoffatomen oder Phenyl substituiert ist,
G
- für ein Sauerstoff- oder ein Schwefelatom steht,
X
- für eine Gruppe der Formel -CH₂-CH₂- oder -CH=CH-steht
und
R
- für eine Gruppe der Formel 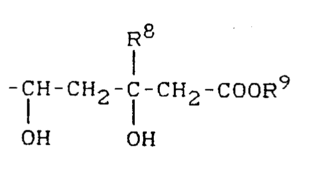 oder 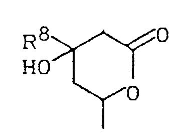 steht,
worin
R⁸
- Wasserstoff
R⁹
- Wasserstoff oder geradkettiges oder verzweigtes Alkyl mit bis zu 8 Kohlenstoffatomen oder Benzyl bedeutet, oder
- Phenyl oder ein Kation bedeutet,
oder
D
- für die Gruppe der Formel -X-R steht,
worin
X und R die oben angegebene Bedeutung haben
und deren Salze.

2. 2-Pyridone und Pyrid-2-thione nach Anspruch 1, worin
A
- für Oxiranyl, Pyridyl, Pyrimidyl, Chinolyl oder Isochinolyl steht, das gegebenenfalls bis zu 3-fach gleich oder verschieden durch Fluor, Chlor, Trifluormethyl, geradkettiges oder verzweigtes Alkyl, Alkylthio, Alkylsulfonyl, Alkoxy oder Alkoxycarbonyl mit jeweils bis zu 4 Kohlenstoffatomen, Phenyl, Phenylthio, Phenylsulfonyl oder durch eine Gruppe der Formel -NR¹R² substituiert ist,
worin
R¹ und R² gleich oder verschieden sind und
- Wasserstoff, Phenyl, Phenylsulfonyl, geradkettiges oder verzweigtes Alkyl oder Alkylsulfonyl mit bis zu 4 Kohlenstoffatomen, Benzyl oder Benzylsulfonyl bedeuten,
- eine Gruppe der Formel -COR³ bedeuten,
worin
R³
- geradkettiges oder verzweigtes Alkyl oder Alkoxy mit bis zu 4 Kohlenstoffatomen oder Phenyl bedeutet,
oder
R¹ und R² gemeinsam mit dem Stickstoffatom einen 5- bis 7-gliedrigen Ring bilden, der durch geradkettiges oder verzweigtes Alkyl mit bis zu 4 Kohlenstoffatomen substituiert sein kann,
- für Phenyl steht, das gegebenenfalls bis zu 3-fach gleich oder verschieden substituiert ist durch geradkettiges oder verzweigtes Alkyl, Alkylthio, Alkylsulfonyl, Alkoxy oder Alkoxycarbonyl mit jeweils bis zu 6 Kohlenstoffatomen, welche ihrerseits durch Hydroxy, Methoxy, Ethoxy, Propoxy, Phenyl oder durch eine Gruppe der Formel -NR¹R² substituiert sein können,
oder durch Phenyl, Phenyloxy, Fluor, Chlor, Nitro, Cyano, Trifluormethyl, Benzyloxy oder eine Gruppe der Formel -NR¹R² substituiert ist,
worin
R¹ und R² die oben angegebene Bedeutung haben,
B
- für Cyclopropyl, Cyclobutyl, Cyclopentyl oder Cyclohexyl steht,
- für geradkettiges oder verzweigtes Alkyl mit bis zu 8 Kohlenstoffatomen steht, das gegebenenfalls durch Fluor, Chlor, Cyano, Azido, Alkoxy mit bis zu 6 Kohlenstoffatomen, Phenyl oder Phenoxy substituiert ist, die ihrerseits durch Fluor, Chlor, Cyano, geradkettiges oder verzweigtes Alkyl oder Alkoxy mit bis zu 4 Kohlenstoffatomen oder durch eine Gruppe der Formel -NR¹R² oder -COR³ substituiert sind,
- für Phenyl steht, das durch Fluor, Chlor, Nitro, geradkettiges oder verzweigtes Alkyl, Alkoxy oder Alkoxycarbonyl mit jeweils bis zu 4 Kohlenstoffatomen oder Amino substituiert ist,
D und E gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclopentyl oder Cyclohexyl stehen,
- für geradkettiges oder verzweigtes Alkyl oder Alkenyl mit jeweils bis zu 8 Kohlenstoffatomen oder Imino stehen, die gegebenenfalls durch Fluor, Chlor, Azido, 2,5-Dioxo-tetrahydropyrryl, Phenyl, Pyrrolidinyl, Morpholino oder Morpholino-N-oxid substituiert ist, oder durch eine Gruppe der Formel -NR¹R², -OR⁴, -COR⁵ oder S(O)ₙ-R⁶ substituiert sind,
worin
R¹ und R² die oben angegebene Bedeutung haben,
R⁴
- Wasserstoff oder
- geradkettiges oder verzweigtes Alkyl mit bis zu 6 Kohlenstoffatomen bedeutet, das gegebenenfalls durch Hydroxy, Dimethyltert.-butylsilyl, Fluor, Chlor oder durch Phenyl substituiert ist, welches seinerseits durch Fluor, Chlor, Hydroxy oder Amino substituiert sein kann,
- Trialkylsilyl mit bis zu 6 Kohlenstoffatomen im gesamten Alkylteil, Tetrahydropyranyl oder 2,5-Dioxo-tetra-hydropyrryl bedeutet,
- Cyclopropyl, Cyclopentyl, Cyclohexyl oder Phenyl bedeutet, oder
- eine Gruppe der Formel -COR⁷ bedeutet,
worin
R⁷
- geradkettiges oder verzweigtes Alkyl mit bis zu 4 Kohlenstoffatomen, Phenyl oder eine Gruppe der Formel -NR¹R² bedeutet,
R⁵
- Wasserstoff, geradkettiges oder verzweigtes Alkyl mit bis zu 6 Kohlenstoffatomen bedeutet, das gegebenenfalls durch Hydroxy, Phenyl, Fluor oder Chlor substituiert ist,
- Phenyl, Pyrryl, Pyrrolidinyl oder Morpholino bedeutet, oder
- eine Gruppe der Formel -NR¹R² oder -OR⁴ bedeutet,
n
- eine Zahl 0 oder 2 bedeutet,
R⁶
- geradkettiges oder verzweigtes Alkyl mit bis zu 6 Kohlenstoffatomen bedeutet, das durch Fluor, Chlor, Hydroxy, Phenyl oder durch eine Gruppe der Formel -NR¹R² substituiert sein kann,
- Phenyl bedeutet, das durch Fluor, Chlor, Hydroxy, Cyano, Nitro oder Amino substituiert sein kann,
- eine Gruppe der Formel -NR¹R² bedeutet, wenn n für die Zahl 2 steht,
oder
D und E gleich oder verschieden sind und
- für eine Gruppe der Formel -NR¹R², -OR⁴ oder -COR⁵ stehen,
worin
R¹, R², R⁴ und R⁵ die oben angegebene Bedeutung haben,
oder
D oder E gemeinsam mit B einen 5- bis 7-gliedrigen, gesättigten oder ungesättigten Ring bilden, der gegebenenfalls durch Methyl, Ethyl, Propyl, Isopropyl, Butyl, tert.Butyl oder Phenyl substituiert ist,
G
- für ein Sauerstoff- oder Schwefelatom steht,
X
- für eine Gruppe -CH=CH- steht
und
R
- für eine Gruppe der Formel 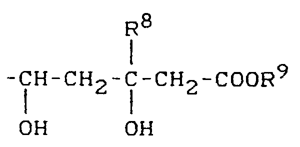 oder 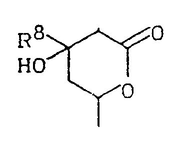 steht,
worin
R⁸
- Wasserstoff,
und
R⁹
- Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet
oder

3. Verfahren zur Herstellung von substituierten 2-Pyridonen und Pyrid-2-thionen nach den Ansprüchen 1 und 2, dadurch gekennzeichnet, daß man
Ketone der allgemeinen Formel (VIII) 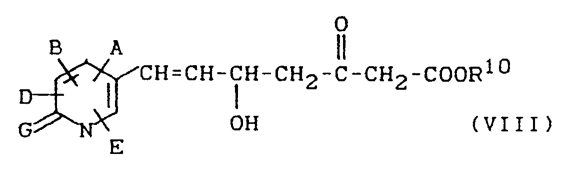 in welcher
A, B, D, E und G die oben angegebene Bedeutung haben,
und
R¹⁰ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = -CH₂-CH₂-) die Ethenverbindungen (X = -CH=CH-) nach üblichen Methoden hydriert,
und gegebenenfalls Isomeren trennt.

4. 2-Pyridone und Pyrid-2-thione nach Anspruch 1 zur Bekämpfung von Krankheiten.

5. Arzneimittel, enthaltend mindestens ein 2-Pyridon oder Pyrid-2-thion nach Anspruch 1.

6. Verfahren zur Herstellung eines Arzneimittels nach Anspruch 6, dadurch gekennzeichnet, daß man 2-Pyridone oder Pyrid-2-thione nach Anspruch 1 gegebenenfalls mit Hilfe von üblichen Hilfs- und Trägerstoffen in eine geeignete Applikationsform bringt.

7. Verwendung der 2-Pyridone und Pyrid-2-thione nach Anspruch 1 zur Herstellung von Arzneimitteln.

8. Verwendung der 2-Pyridone und Pyrid-2-thione nach Anspruch 1 zur Herstellung von Arzneimitteln zur Behandlung von Hyperlipoproteinämie, Lipoproteinämie oder Arteriosklerose.

9. Ketone der allgemeinen Formel (VIII) 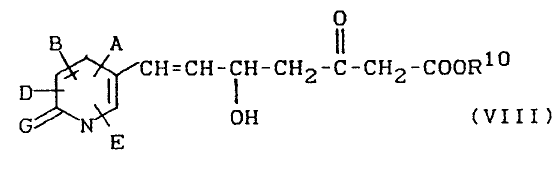 in welcher
A
- für Oxiranyl, Thienyl, Furyl, Pyridyl, Pyrimidyl, Pyrazinyl, Indolyl, Chinolyl, Isochinolyl, Benzothiazolyl oder Benzimidazolyl steht, das gegebenenfalls bis zu 4-fach gleich oder verschieden durch Fluor, Chlor, Brom, Trifluormethyl, Trifluormethylthio, Trifluormethoxy, geradkettiges oder verzweigtes Alkyl, Alkylthio, Alkylsulfonyl, Alkoxy oder Alkoxycarbonyl mit jeweils bis zu 6 Kohlenstoffatomen, Phenyl, Phenylthio, Phenylsulfonyl oder durch eine Gruppe der Formel -NR¹R² substituiert ist,
worin
R¹ und R² gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel -COR³ bedeuten,
worin
R³
- geradkettiges oder verzweigtes Alkyl oder Alkoxy mit bis zu 6 Kohlenstoffatomen oder Phenyl bedeutet,
oder
R¹ und R² gemeinsam mit dem Stickstoffatom einen 5- bis 7-gliedrigen Ring bilden, der durch geradkettiges oder verzweigtes Alkyl mit bis zu 6 Kohlenstoffatomen substituiert sein kann,
- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy, Alkoxy mit bis zu 4 Kohlenstoffatomen, Phenyl oder durch eine Gruppe der Formel -NR¹R² substituiert sein können,
oder durch Phenyl, Phenyloxy, Phenylthio, Phenylsulfonyl, Fluor, Chlor, Brom, Nitro, Cyano, Trifluormethyl, Trifluormethoxy, Trifluormethylthio, Benzyloxy oder durch eine Gruppe der Formel -NR¹R² substituiert ist,
B
- für Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, Cyano, Azido, Trifluormethyl, Trifluormethoxy, Trifluormethylthio, Trifluormethylsulfonyl, Alkoxy mit bis zu 8 Kohlenstoffatomen oder durch Phenyl, Phenyloxy, oder Phenylthio, Thienyl, Furyl, Pyridyl, Pyrimidyl oder Chinolyl substituiert ist, welches seinerseits bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein kann, oder
durch eine Gruppe der Formel -NR¹R² oder -COR³ substituiert ist
- für Phenyl steht, das gegebenenfalls bis zu 2-fach gleich oder verschieden durch Fluor, Chlor, Brom, Cyano, Nitro, Trifluormethyl, geradkettiges oder verzweigtes Alkyl, Alkoxy oder Alkoxycarbonyl mit jeweils bis zu 6 Kohlenstoffatomen oder Amino substituiert ist,
D und E gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclobutyl, Cyclopentyl oder Cyclohexyl stehen,
- für geradkettiges oder verzweigtes Alkyl oder Alkenyl mit jeweils bis zu 10 Kohlenstoffatomen oder Imino stehen, die gegebenenfalls durch Fluor, Chlor, Brom, Azido, 2,5-Dioxotetrahydropyrryl, Phenyl, Pyrimidyl, Pyrryl, Pyrrolidinyl, Morpholino oder Morpholino-N-oxid substituiert ist, oder durch eine Gruppe der Formel NR¹R², -OR⁴, -COR⁵ oder
-S(O)ₙ-R⁶ substituiert sind,
worin
R¹ und R² die oben angegebene Bedeutung haben,
R⁴
- Wasserstoff oder
- geradkettiges oder verzweigtes Alkyl mit bis zu 8 Kohlenstoffatomen bedeutet, das gegebenenfalls durch Hydroxy, Trialkylsilyl mit bis zu 8 Kohlenstoffatomen im gesamten Alkylteil, Fluor, Chlor, Brom oder durch Phenyl substituiert ist, welches seinerseits durch Fluor, Chlor, Brom, Cyano, Nitro, Hydroxy, geradkettiges oder verzweigtes Alkyl, Alkoxy oder Alkoxycarbonyl mit jeweils bis zu 6 Kohlenstoffatomen oder Amino substituiert sein kann,
- Trialkylsilyl mit bis zu 8 Kohlenstoffatomen im gesamten Alkylteil, Tetrahydropyranyl oder 2,5-Dioxo-tetrahydropyrryl bedeutet,
- Cyclopropyl, Cyclobutyl, Cyclopentyl, Cyclohexyl oder Phenyl bedeutet, welches seinerseits durch Fluor, Chlor, Brom, Cyano, Nitro oder Amino substituiert sein kann, oder
- eine Gruppe der Formel -COR⁷ bedeutet,
worin
R⁷
- geradkettiges oder verzweigtes Alkyl mit bis zu 6 Kohlenstoffatomen, Phenyl oder eine Gruppe der Formel -NR¹R² bedeutet,
R⁵
- Wasserstoff, geradkettiges oder verzweigtes Alkyl mit bis zu 8 Kohlenstoffatomen bedeutet, das gegebenenfalls durch Hydroxy, Phenyl, Fluor, Chlor, Brom oder Cyano substituiert ist,
- Phenyl, Naphthyl, Pyrryl, Pyrimidyl, Pyridyl, Pyrrolidinyl oder Morpholino bedeutet, welches seinerseits durch Fluor, Chlor, Brom, Amino, Hydroxy, Nitro oder Cyano substituiert sein kann, oder
- eine Gruppe der Formel -NR¹R² oder -OR⁴ bedeutet,
n
- eine Zahl 0 oder 2 bedeutet,
R⁶
- geradkettiges oder verzweigtes Alkyl mit bis zu 8 Kohlenstoffatomen bedeutet, das durch Fluor, Chlor, Brom, Hydroxy, Phenyl oder durch eine Gruppe der Formel -NR¹R² substituiert sein kann,
- Phenyl bedeutet, das durch Fluor, Chlor, Brom, Hydroxy, Cyano, Nitro oder Amino substituiert sein kann, oder
- eine Gruppe der Formel -NR¹R² bedeutet, wenn n für die Zahl 2 steht,
oder
D und E gleich oder verschieden sind und
- für eine Gruppe der Formel -NR¹R², -OR⁴ oder -COR⁵ stehen,
worin
R¹, R², R⁴ und R⁵ die oben angegebene Bedeutung haben,
D oder E gemeinsam mit B einer 5- bis 7-gliedrigen, gesättigten oder ungesättigten Ring bilden, der gegebenenfalls durch geradkettiges oder verzweigtes Alkyl mit bis zu 6 Kohlenstoffatomen oder Phenyl substituiert ist,
G
- für ein Sauerstoff- oder ein Schwefelatom steht,
oder
D
- für die Gruppe der Formel -X-R steht,
X
- für eine Gruppe der Formel -CH₂-CH₂- oder -CH=CH-steht
und
R
- für eine Gruppe der Formel 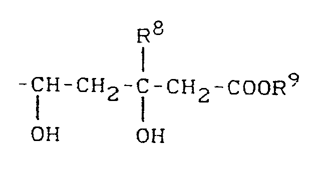 oder 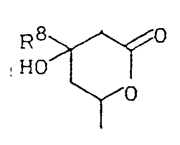 steht,
worin
R⁸
- Wasserstoff
R⁹
- Wasserstoff oder geradkettiges oder verzweigtes Alkyl mit bis zu 8 Kohlenstoffatomen oder Benzyl bedeutet, oder
- Phenyl oder ein Kation bedeutet,
und deren Salze.

10. Verfahren zur Herstellung von Ketonen nach Anspruch 9 dadurch gekennzeichnet, daß man Aldehyde der allgemeinen Formel (IX) 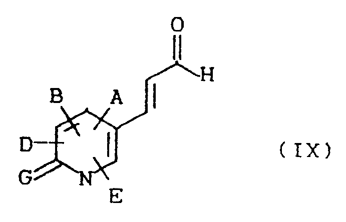 in welcher
A, B, D, E und G die oben angegebene Bedeutung haben,
in inerten Lösemitteln mit Acetessigester der allgemeinen Formel (X) 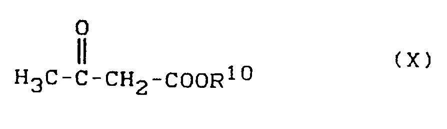 in welcher
R¹⁰ die oben angegebene Bedeutung hat,
in Anwesenheit von Basen umsetzt.

## Patentansprüche (Patentansprüche für folgende(n) Vertragsstaat(en): ES)

1. Verfahren zur Herstellung von substituierten 2-Pyridonen und Pyrid-2-thionen der allgemeinen Formeln (Ia) und (Ib) 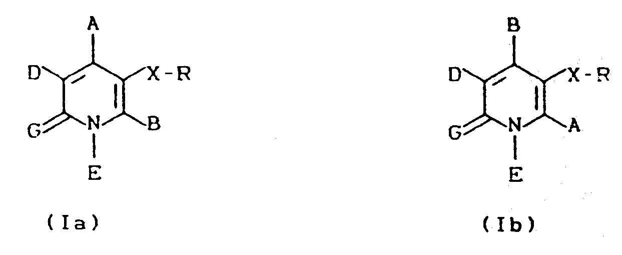 in welcher
A
- für Oxiranyl, Thienyl, Furyl, Pyridyl, Pyrimidyl, Pyrazinyl, Indolyl, Chinolyl, Isochinolyl, Benzothiazolyl oder Benzimidazolyl steht, das gegebenenfalls bis zu 4-fach gleich oder verschieden durch Fluor, Chlor, Brom, Trifluormethyl, Trifluormethylthio, Trifluormethoxy, geradkettiges oder verzweigtes Alkyl, Alkylthio, Alkylsulfonyl, Alkoxy oder Alkoxycarbonyl mit jeweils bis zu 6 Kohlenstoffatomen, Phenyl, Phenylthio, Phenylsulfonyl oder durch eine Gruppe der Formel -NR¹R² substituiert ist,
worin
R¹ und R² gleich oder verschieden sind und
- Wasserstoff, Phenyl, Phenylsulfonyl, geradkettiges oder verzweigtes Alkyl oder Alkylsulfonyl mit bis zu 6 Kohlenstoffatomen, Benzyl oder Benzylsulfonyl bedeuten, oder
- eine Gruppe der Formel -COR³ bedeuten,
worin
R³
- geradkettiges oder verzweigtes Alkyl oder Alkoxy mit bis zu 6 Kohlenstoffatomen oder Phenyl bedeutet,
oder
R¹ und R² gemeinsam mit dem Stickstoffatom einen 5- bis 7-gliedrigen Ring bilden, der durch geradkettiges oder verzweigtes Alkyl mit bis zu 6 Kohlenstoffatomen substituiert sein kann,
- für Phenyl oder Naphthyl steht, das gegebenenfalls bis zu 4-fach gleich oder verschieden substituiert ist durch geradkettiges oder verzweigtes Alkyl, Alkylthio, Alkylsulfonyl, Alkoxy oder Alkoxycarbonyl mit jeweils bis zu 8 Kohlenstoffatomen, welche ihrerseits durch Hydroxy, Alkoxy mit bis zu 4 Kohlenstoffatomen, Phenyl oder durch eine Gruppe der Formel -NR¹R² substituiert sein können,
oder durch Phenyl, Phenyloxy, Phenylthio, Phenylsulfonyl, Fluor, Chlor, Brom, Nitro, Cyano, Trifluormethyl, Trifluormethoxy, Trifluormethylthio, Benzyloxy oder durch eine Gruppe der Formel -NR¹R² substituiert ist,
B
- für Cyclopropyl, Cyclobutyl, Cyclopentyl oder Cyclohexyl steht,
- für geradkettiges oder verzweigtes Alkyl mit bis zu 10 Kohlenstoffatomen steht, das gegebenenfalls durch Fluor, Chlor, Brom, Cyano, Azido, Trifluormethyl, Trifluormethoxy, Trifluormethylthio, Trifluormethylsulfonyl, Alkoxy mit bis zu 8 Kohlenstoffatomen oder durch Phenyl, Phenyloxy, oder Phenylthio, Thienyl, Furyl, Pyridyl, Pyrimidyl oder Chinolyl substituiert ist, welches seinerseits bis zu 2-fach gleich oder verschieden durch Fluor, Chlor, Brom, Cyano, Trifluormethyl, Trifluormethoxy, geradkettiges oder verzweigtes Alkyl, Alkoxy, Alkylthio oder Alkylsulfonyl mit jeweils bis zu 6 Kohlenstoffatomen substituiert sein kann, oder
durch eine Gruppe der Formel -NR¹R² oder -COR³ substituiert ist
- für Phenyl steht, das gegebenenfalls bis zu 2-fach gleich oder verschieden durch Fluor, Chlor, Brom, Cyano, Nitro, Trifluormethyl, geradkettiges oder verzweigtes Alkyl, Alkoxy oder Alkoxycarbonyl mit jeweils bis zu 6 Kohlerstoffatomen oder Amino substituiert ist,
D und E gleich oder verschieden sind und die oben angegebene Bedeutung von A haben und mit dieser gleich oder verschieden sind, oder
- für Wasserstoff, Nitro oder Cyano stehen,
- für Cyclopropyl, Cyclobutyl, Cyclopentyl oder Cyclohexyl stehen,
- für geradkettiges oder verzweigtes Alkyl oder Alkenyl mit jeweils bis zu 10 Kohlenstoffatomen oder Imino stehen, die gegebenenfalls durch Fluor, Chlor, Brom, Azido, 2,5-Dioxotetrahydropyrryl, Phenyl, Pyrimidyl, Pyrryl, Pyrrolidinyl, Morpholino oder Morpholino-N-oxid substituiert ist, oder durch eine Gruppe der Formel NR¹R², -OR⁴, -COR⁵ oder
-S(O)ₙ-R⁶ substituiert sind,
worin
R¹ und R² die oben angegebene Bedeutung haben,
R⁴
- Wasserstoff oder
- geradkettiges oder verzweigtes Alkyl mit bis zu 8 Kohlenstoffatomen bedeutet, das gegebenenfalls durch Hydroxy, Trialkylsilyl mit bis zu 8 Kohlenstoffatomen im gesamten Alkylteil, Fluor, Chlor, Brom oder durch Phenyl substituiert ist, welches seinerseits durch Fluor, Chlor, Brom, Cyano, Nitro, Hydroxy, geradkettiges oder verzweigtes Alkyl, Alkoxy oder Alkoxycarbonyl mit jeweils bis zu 6 Kohlenstoffatomen oder Amino substituiert sein kann,
- Trialkylsilyl mit bis zu 8 Kohlenstoffatomen im gesamten Alkylteil, Tetrahydropyranyl oder 2,5-Dioxo-tetrahydropyrryl bedeutet,
- Cyclopropyl, Cyclobutyl, Cyclopentyl, Cyclohexyl oder Phenyl bedeutet, welches seinerseits durch Fluor, Chlor, Brom, Cyano, Nitro oder Amino substituiert sein kann, oder
- eine Gruppe der Formel -COR⁷ bedeutet,
worin
R⁷
- geradkettiges oder verzweigtes Alkyl mit bis zu 6 Kohlenstoffatomen, Phenyl oder eine Gruppe der Formel -NR¹R² bedeutet,
R⁵
- Wasserstoff, geradkettiges oder verzweigtes Alkyl mit bis zu 8 Kohlenstoffatomen bedeutet, das gegebenenfalls durch Hydroxy, Phenyl, Fluor, Chlor, Brom oder Cyano substituiert ist,
- Phenyl, Naphthyl, Pyrryl, Pyrimidyl, Pyridyl, Pyrrolidinyl oder Morpholino bedeutet, welches seinerseits durch Fluor, Chlor, Brom, Amino, Hydroxy, Nitro oder Cyano substituiert sein kann, oder
- eine Gruppe der Formel -NR¹R² oder -OR⁴ bedeutet,
n
- eine Zahl 0 oder 2 bedeutet,
R⁶
- geradkettiges oder verzweigtes Alkyl mit bis zu 8 Kohlenstoffatomen bedeutet, das durch Fluor, Chlor, Brom, Hydroxy, Phenyl oder durch eine Gruppe der Formel -NR¹R² substituiert sein kann,
- Phenyl bedeutet, das durch Fluor, Chlor, Brom, Hydroxy, Cyano, Nitro oder Amino substituiert sein kann, oder
- eine Gruppe der Formel -NR¹R² bedeutet, wenn n für die Zahl 2 steht,
oder
D und E gleich oder verschieden sind und
- für eine Gruppe der Formel -NR¹R², -OR⁴ oder -COR⁵ stehen,
worin
R¹, R², R⁴ und R⁵ die oben angegebene Bedeutung haben,
D oder E gemeinsam mit B einen 5- bis 7-gliedrigen, gesättigten oder ungesättigten Ring bilden, der gegebenenfalls durch geradkettiges oder verzweigtes Alkyl mit bis zu 6 Kohlenstoffatomen oder Phenyl substituiert ist,
G
- für ein Sauerstoff- oder ein Schwefelatom steht,
X
- für eine Gruppe der Formel -CH₂-CH₂- oder -CH=CH-steht
und
R
- für eine Gruppe der Formel 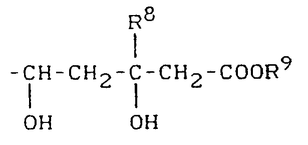 oder 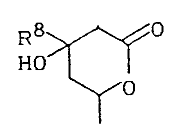 steht,
worin
R⁸
- Wasserstoff
R⁹
- Wasserstoff oder geradkettiges oder verzweigtes Alkyl mit bis zu 8 Kohlenstoffatomen oder Benzyl bedeutet, oder
- Phenyl oder ein Kation bedeutet,
oder
D
- für die Gruppe der Formel -X-R steht,
worin
X und R die oben angegebene Bedeutung haben und deren Salze, dadurch gekennzeichnet, daß man
Ketone der allgemeinen Formel (VIII) 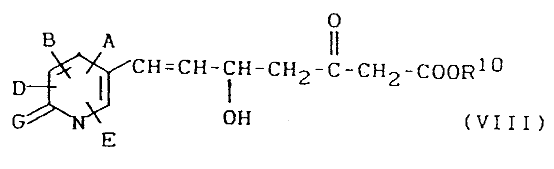 in welcher
A, B, D, E und G die oben angegebene Bedeutung haben,
und
R¹⁰
- für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = -CH₂-CH₂-) die Ethenverbindungen (X = -CH=CH-) nach üblichen Methoden hydriert,
und gegebenenfalls Isomeren trennt.

2. Verfahren zur Herstellung von Ketonen der Formel 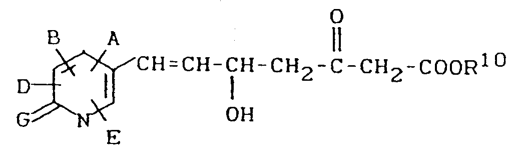 in welcher
A, B, D, E, G und R¹⁰ die in Anspruch 1 angegebene Bedeutung haben und
dadurch gekennzeichnet, daß man Aldehyde der allgemeinen Formel (IX) 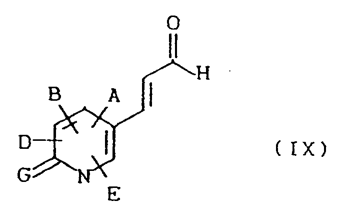 in welcher
A, B, D, E und G die oben angegebene Bedeutung haben,
in inerten Lösemitteln mit Acetessigester der allgemeinen Formel (X) 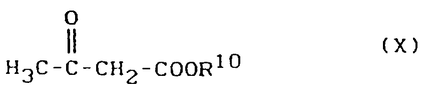 in welcher
R¹⁰ die oben angegebene Bedeutung hat,
in Anwesenheit von Basen umsetzt.

## Claims (Claims for the following Contracting State(s): AT, BE, CH, DE, FR, GB, GR, IT, LI, LU, NL, SE)

1. 2-Pyridones and pyrid-2-thiones of the general formulae (Ia) and (Ib) 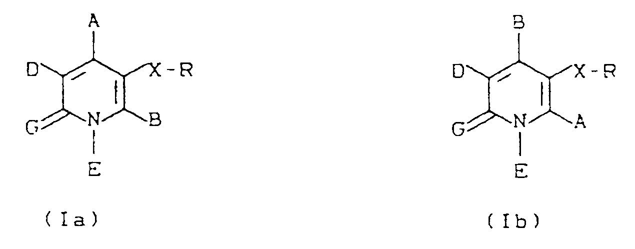 in which
A
- represents oxiranyl, thienyl, furyl, pyridyl, pyrimidyl, pyrazinyl, indolyl, quinolyl, isoquinolyl, benzothiazolyl or benzimidazolyl, each of which is optionally monosubstituted to tetrasubstituted by identical or different substituents from the series comprising fluorine, chlorine, bromine, trifluoromethyl, trifluoromethylthio, trifluoromethoxy, straight-chain or branched alkyl, alkylthio, alkylsulphonyl, alkoxy or alkoxycarbonyl each having up to 6 carbon atoms, phenyl, phenylthio, phenylsulphonyl or by a group of the formula -NR¹R²,
in which
R¹ and R² are identical or different and
- denote hydrogen, phenyl, phenylsulphonyl, straight-chain or branched alkyl or alkylsulphonyl having up to 6 carbon atoms, benzyl or benzylsulphonyl, or
- denote a group of the formula -COR³,
in which
R³
- denotes straight-chain or branched alkyl or alkoxy having up to 6 carbon atoms or phenyl,
or
R¹ and R², together with the nitrogen atom, form a 5- to 7-membered ring which may be substituted by straight-chain or branched alkyl having up to 6 carbon atoms,
- represents phenyl or naphthyl, each of which is optionally monosubstituted to tetrasubstituted by identical or different substituents from the series comprising straight-chain or branched alkyl, alkylthio, alkylsulphonyl, alkoxy or alkoxycarbonyl each having up to 8 carbon atoms, which may in turn be substituted by hydroxyl, alkoxy having up to 4 carbon atoms, phenyl or by a group of the formula -NR¹R²,
or by phenyl, phenyloxy, phenylthio, phenylsulphonyl, fluorine, chlorine, bromine, nitro, cyano, trifluoromethyl, trifluoromethoxy, trifluoromethylthio, benzyloxy or by a group of the formula -NR¹R²,
B
- represents cyclopropyl, cyclobutyl, cyclopentyl or cyclohexyl,
- represents straight-chain or branched alkyl having up to 10 carbon atoms, which is optionally substituted by fluorine, chlorine, bromine, cyano, azido, trifluoromethyl, trifluoromethoxy, trifluoromethylthio, trifluoromethylsulphonyl, alkoxy having up to 8 carbon atoms or by phenyl, phenyloxy, or phenylthio, thienyl, furyl, pyridyl, pyrimidyl or quinolyl which may in turn be monosubstituted or disubstituted by identical or different substituents from the series comprising fluorine, chlorine, bromine, cyano, trifluoromethyl, trifluoromethoxy, straight-chain or branched alkyl, alkoxy, alkylthio or alkylsulphonyl each having up to 6 carbon atoms, or by a group of the formula -NR¹R² or -COR³,
- represents phenyl which is optionally substituted by substituents from the series comprising fluorine, chlorine, bromine, cyano, nitro, trifluoromethyl, straight-chain or branched alkyl, alkoxy or alkoxycarbonyl each having up to 6 carbon atoms or amino,
D and E are identical or different and have the above-mentioned meaning of A and are identical or different to this, or
- represent hydrogen, nitro or cyano,
- represent cyclopropyl, cyclobutyl, cyclopentyl or cyclohexyl,
- represent straight-chain or branched alkyl or alkenyl each having up to 10 carbon atoms or nitrilo which is optionally substituted by fluorine, chlorine, bromine, azido, 2,5-dioxo-tetrahydropyrryl, phenyl, pyrimidyl, pyrryl, pyrrolidinyl, morpholino or morpholino-N-oxide, or by a group of the formula NR¹R², -OR⁴, -COR⁵ or -S(O)ₙR-⁶,
in which
R¹ and R² have the abovementioned meaning,
R⁴
- denotes hydrogen or
- denotes straight-chain or branched alkyl having up to 8 carbon atoms, which is optionally substituted by hydroxyl, trialkylsilyl having up to 8 carbon atoms in the alkyl moiety, fluorine, chlorine, bromine or by phenyl which may in turn be substituted by fluorine, chlorine, bromine, cyano, nitro, hydroxyl, straight-chain or branched alkyl, alkoxy or alkoxycarbonyl each having up to 6 carbon atoms or amino,
- denotes trialkylsilyl having up to 8 carbon atoms, tetrahydropyranyl or 2,5-dioxo-tetrahydropyrryl,
- denotes cyclopropyl, cyclobutyl, cyclopentyl, cyclohexyl or phenyl which may in turn be substituted by fluorine, chlorine, bromine, cyano, nitro or amino, or
- denotes a group of the formula -COR⁷,
in which
R⁷
- denotes straight-chain or branched alkyl having up to 6 carbon atoms, phenyl or a group of the formula - NR¹R²,
R⁵
- denotes hydrogen, straight-chain or branched alkyl having up to 8 carbon atoms, which is optionally substituted by hydroxyl, phenyl, fluorine, chlorine, bromine or cyano,
- denotes phenyl, naphthyl, pyrryl, pyrimidyl, pyridyl, pyrrolidinyl or morpholino which may in turn be substituted by fluorine, chlorine, bromine, amino, hydroxyl, nitro or cyano, or
- denotes a group of the formula - NR¹R² or -OR⁴,
n
- denotes a number 0 or 2,
R⁶
- denotes straight-chain or branched alkyl having up to 8 carbon atoms, which may be substituted by fluorine, chlorine, bromine, hydroxyl, phenyl or by a group of the formula -NR¹R²,
- denotes phenyl which may be substituted by fluorine, chlorine, bromine, hydroxyl, cyano, nitro or amino, or
- denotes a group of the formula - NR¹R² if n represents the number 2,
or
D and E are identical or different and
- represent a group of the formula -NR¹R², -OR⁴ or -COR⁵,
in which
R¹, R², R⁴ and R⁵ have the abovementioned meaning,
or
D and E together with B form a 5- to 7-membered saturated or unsaturated ring which is optionally substituted by straight-chain or branched alkyl having up to 6 carbon atoms or phenyl,
G
- represents an oxygen or a sulphur atom,
X
- represents a group of the formula -CH₂-CH₂- or -CH=CH-
and
R
- represents a group of the formula 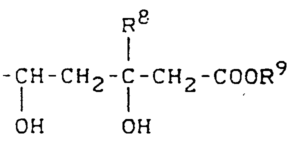 or 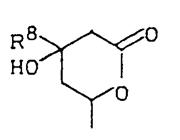
in which
R⁸
- denotes hydrogen
and
R⁹
- denotes hydrogen or straight-chain or branched alkyl having up to 8 carbon atoms or benzyl, or
- denotes phenyl or a cation,
or
D
- represents the group of the formula -X-R,
in which
X and R have the abovementioned meaning,
and their salts.

2. 2-Pyridones and pyrid-2-thiones according to Claim 1, in which
A
- represents oxiranyl, pyridyl, pyrimidyl, quinolyl or isoquinolyl, each of which is optionally monosubstituted to trisubstituted by identical or different substituents from the series comprising fluorine, chlorine, trifluoromethyl, straight-chain or branched alkyl, alkylthio, alkylsulphonyl, alkoxy or alkoxycarbonyl each having up to 4 carbon atoms, phenyl, phenylthio, phenylsulphonyl or by a group of the formula -NR¹R²,
in which
R¹ and R² are identical or different and
- denotes hydrogen, phenyl, phenylsulphonyl, straight-chain or branched alkyl or alkylsulphonyl having up to 4 carbon atoms, benzyl or benzylsulphonyl,
- denote a group of the formula -COR³,
in which
R³
- denotes straight-chain or branched alkyl or alkoxy having up to 4 carbon atoms or phenyl,
or
R¹ and R², together with the nitrogen atom, form a 5- to 7-membered ring which may be substituted by straight-chain or branched alkyl having up to 4 carbon atoms,
- represents phenyl which is optionally monosubstituted to trisubstituted by identical or different substituents from the series comprising straight-chain or branched alkyl, alkylthio, alkylsulphonyl, alkoxy or alkoxycarbonyl each having up to 6 carbon atoms, which may in turn be substituted by hydroxyl, methoxy, ethoxy, propoxy, phenyl or by a group of the formula -NR¹R²,
or by phenyl, phenyloxy, fluorine, chlorine, nitro, cyano, trifluoromethyl, benzyloxy or a group of the formula -NR¹R²,
in which
R¹ and R² have the abovementioned meaning,
B
- represents cyclopropyl, cyclobutyl, cyclopentyl or cyclohexyl,
- represents straight-chain or branched alkyl having up to 8 carbon atoms, which is optionally substituted by fluorine, chlorine, cyano, azido, alkoxy having up to 6 carbon atoms, phenyl or phenoxy which is in turn substituted by fluorine, chlorine, cyano, straight-chain or branched alkyl or alkoxy having up to 4 carbon atoms or by a group of the formula -NR¹R² or -COR³,
- represents phenyl which is substituted by fluorine, chlorine, nitro, straight-chain or branched alkyl, alkoxy or alkoxycarbonyl each having up to 4 carbon atoms or amino,
D and E are identical or different and have the abovementioned meaning of A and are identical or different to this, or
- represent hydrogen, nitro or cyano,
- represent cyclopropyl, cyclopentyl or cyclohexyl,
- represent straight-chain or branched alkyl or alkenyl each having up to 8 carbon atoms or nitrilo which is optionally substituted by fluorine, chlorine, azido, 2,5-dioxo-tetrahydropyrryl, phenyl, pyrrolidinyl, morpholino or morpholino-N-oxide, or is substituted by a group of the formula -NR¹R², -OR⁴, -COR⁵ or -S(O)ₙ-R⁶,
in which
R¹ and R² have the abovementioned meaning,
R⁴
- denotes hydrogen or
- denotes straight-chain or branched alkyl having up to 6 carbon atoms, which is optionally substituted by hydroxyl, dimethyl-tert.-butylsilyl, fluorine, chlorine or by phenyl which may in turn be substituted by fluorine, chlorine, hydroxyl or amino,
- denotes trialkylsilyl having up to 6 carbon atoms, tetrahydropyranyl or 2,5-dioxo-tetra-hydropyrryl,
- denotes cyclopropyl, cyclopentyl, cyclohexyl or phenyl, or
- denotes a group of the formula -COR⁷,
in which
R⁷
- denotes straight-chain or branched alkyl having up to 4 carbon atoms, phenyl or a group of the formula
-NR¹R²,
R⁵
- denotes hydrogen, straight-chain or branched alkyl having up to 6 carbon atoms, which is optionally substituted by hydroxyl, phenyl, fluorine or chlorine,
- denotes phenyl, pyrryl, pyrrolidinyl or morpholino, or
- denotes a group of the formula -NR¹R² or - OR⁴,
n
- denotes a number 0 or 2,
R⁶
- denotes straight-chain or branched alkyl having up to 6 carbon atoms, which may be substituted by fluorine, chlorine, hydroxyl, phenyl or by a group of the formula -NR¹R²,
- denotes phenyl which may be substituted by fluorine, chlorine, hydroxyl, cyano, nitro or amino,
- denotes a group of the formula -NR¹R² if n represents the number 2,
or
D and E are identical or different and
- represent a group of the formula -NR¹R², -OR⁴ or -COR⁵,
in which
R¹, R², R⁴ and R⁵ have the abovementioned meaning, or
D and E, together with B, form a 5- to 7-membered, saturated or unsaturated ring which is optionally substituted by methyl, ethyl, propyl, isopropyl, butyl, tert.butyl or phenyl,
G
- represents an oxygen or sulphur atom,
X
- represents a -CH=CH- group
and
R
- represents a group of the formula 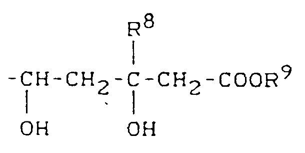 or 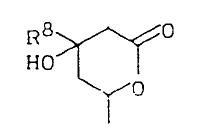
in which
R⁸
- denotes hydrogen,
and
R⁹
- denotes hydrogen, methyl, ethyl, propyl, isopropyl, butyl, isobutyl, tert.butyl or benzyl, or
- denotes a sodium, potassium, calcium, magnesium or ammonium ion
or
D
- represents a group of the formula -X-R,
in which
X and R have the abovementioned meaning,
and their salts.

3. Process for the preparation of substituted 2-pyridones and pyrid-2-thiones according to Claims 1 and 2,
characterized in that
ketones of the general formula (VIII) 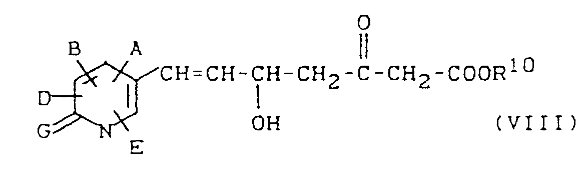 in which
A, B, D, E and G have the abovementioned meaning,
and
R¹⁰ - represents alkyl,
are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds (X = -CH₂-CH₂-) the ethene compounds (X= -CH=CH-) are hydrogenated by customary methods,
and, if appropriate, isomers are separated.

4. 2-Pyridones and pyrid-2-thiones according to Claim 1 for combating diseases.

5. Medicaments, containing at least one 2-pyridone or pyrid-2-thione according to Claim 1.

6. Process for the production of a medicament according to Claim 5, characterized in that 2-pyridones or pyrid-2-thiones according to Claim 1 are brought into a suitable form for administration, if appropriate with the aid of customary auxiliaries and excipients.

7. Use of the 2-pyridones and pyrid-2-thiones according to Claim 1 for the production of medicaments.

8. Use of the 2-pyridones and pyrid-2-thiones according to Claim 1 for the production of medicaments for the treatment of hyperlipoproteinaemia, lipoproteinaemia or arteriosclerosis.

9. Ketones of the general formula (VIII) 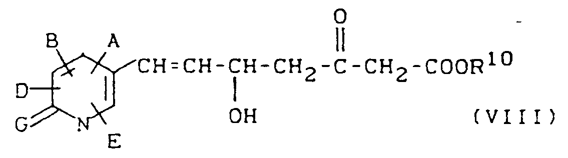 in which
A
- represents oxiranyl, thienyl, furyl, pyridyl, pyrimidyl, pyrazinyl, indolyl, quinolyl, isoquinolyl, benzothiazolyl or benzimidazolyl, each of which is optionally monosubstituted to tetrasubstituted by identical or different substituents from the series comprising fluorine, chlorine, bromine, trifluoromethyl, trifluoromethylthio, trifluoromethoxy, straight-chain or branched alkyl, alkylthio, alkylsulphonyl, alkoxy or alkoxycarbonyl each having up to 6 carbon atoms, phenyl, phenylthio, phenylsulphonyl or by a group of the formula -NR¹R²,
in which
R¹ and R² are identical or different and
- denote hydrogen, phenyl, phenylsulphonyl, straight-chain or branched alkyl or alkylsulphonyl having up to 6 carbon atoms, benzyl or benzylsulphonyl, or
- denotes a group of the formula -COR³,
in which
R³
- denotes straight-chain or branched alkyl or alkoxy having up to 6 carbon atoms or phenyl,
or
R¹ and R², together with the nitrogen atom, form a 5- to 7-membered ring which may be substituted by straight-chain or branched alkyl having up to 6 carbon atoms,
- represents phenyl or naphthyl, each of which is optionally monosubstituted to tetrasubstituted by identical or different substituents from the series comprising straight-chain or branched alkyl, alkylthio, alkylsulphonyl, alkoxy or alkoxycarbonyl each having up to 8 carbon atoms, which may in turn be substituted by hydroxyl, alkoxy having up to 4 carbon atoms, phenyl or by a group of the formula -NR¹R²,
or by phenyl, phenyloxy, phenylthio, phenylsulphonyl, fluorine, chlorine, bromine, nitro, cyano, trifluoromethyl, trifluoromethoxy, trifluoromethylthio, benzyloxy or by a group of the formula -NR¹R²,
B
- represents cyclopropyl, cyclobutyl, cyclopentyl or cyclohexyl,
- represents straight-chain or branched alkyl having up to 10 carbon atoms, which is optionally substituted by fluorine, chlorine, bromine, cyano, azido, trifluoromethyl, trifluoromethoxy, trifluoromethylthio, trifluoromethylsulphonyl, alkoxy having up to 8 carbon atoms or by phenyl, phenyloxy, or phenylthio, thienyl, furyl, pyridyl, pyrimidyl or quinolyl which may in turn be monosubstituted or disubstituted by identical or different substituents from the series comprising fluorine, chlorine, bromine, cyano, trifluoromethyl, trifluoromethoxy, straight-chain or branched alkyl, alkoxy, alkylthio or alkylsulphonyl each having up to 6 carbon atoms, or by a group of the formula -NR¹R² or -COR³,
- represents phenyl which is optionally substituted by substituents from the series comprising fluorine, chlorine, bromine, cyano, nitro, trifluoromethyl, straight-chain or branched alkyl, alkoxy or alkoxycarbonyl each having up to 6 carbon atoms or amino,
D and E are identical or different and have the above-mentioned meaning of A and are identical or different to this, or
- represent hydrogen, nitro or cyano,
- represent cyclopropyl, cyclobutyl, cyclopentyl or cyclohexyl,
- represent straight-chain or branched alkyl or alkenyl each having up to 10 carbon atoms or nitrilo which is optionally substituted by fluorine, chlorine, bromine, azido, 2,5-dioxo-tetrahydropyrryl, phenyl, pyrimidyl, pyrryl, pyrrolidinyl, morpholino or morpholino-N-oxide, or by a group of the formula NR¹R², -OR⁴, -COR⁵ or -S(O)ₙR-⁶,
in which
R¹ and R² have the abovementioned meaning,
R⁴
- denotes hydrogen or
- denotes straight-chain or branched alkyl having up to 8 carbon atoms, which is optionally substituted by hydroxyl, trialkylsilyl having up to 8 carbon atoms in the alkyl moiety, fluorine, chlorine, bromine or by phenyl which may in turn be substituted by fluorine, chlorine, bromine, cyano, nitro, hydroxyl, straight-chain or branched alkyl, alkoxy or alkoxycarbonyl each having up to 6 carbon atoms or amino,
- denotes trialkylsilyl having up to 8 carbon atoms, tetrahydropyranyl or 2,5-dioxo-tetrahydropyrryl,
- denotes cyclopropyl, cyclobutyl, cyclopentyl, cyclohexyl or phenyl which may in turn be substituted by fluorine, chlorine, bromine, cyano, nitro or amino, or
- denotes a group of the formula -COR⁷,
in which
R⁷
- denotes straight-chain or branched alkyl having up to 6 carbon atoms, phenyl or a group of the formula - NR¹R²,
R⁵
- denotes hydrogen, straight-chain or branched alkyl having up to 8 carbon atoms, which is optionally substituted by hydroxyl, phenyl, fluorine, chlorine, bromine or cyano,
- denotes phenyl, naphthyl, pyrryl, pyrimidyl, pyridyl, pyrrolidinyl or morpholino which may in turn be substituted by fluorine, chlorine, bromine, amino, hydroxyl, nitro or cyano, or
- denotes a group of the formula - NR¹R² or -OR⁴,
n
- denotes a number 0 or 2,
R⁶
- denotes straight-chain or branched alkyl having up to 8 carbon atoms, which may be substituted by fluorine, chlorine, bromine, hydroxyl, phenyl or by a group of the formula -NR¹R²,
- denotes phenyl which may be substituted by fluorine, chlorine, bromine, hydroxyl, cyano, nitro or amino, or
- denotes a group of the formula - NR¹R² if n represents the number 2,
or
D and E are identical or different and
- represent a group of the formula -NR¹R², -OR⁴ or -COR⁵,
in which
R¹, R², R⁴ and R⁵ have the abovementioned meaning,
or
D and E together with B form a 5- to 7-membered saturated or unsaturated ring which is optionally substituted by straight-chain or branched alkyl having up to 6 carbon atoms or phenyl,
G
- represents an oxygen or a sulphur atom,
or
D
- represents the group of the formula -X-R,
X
- represents a group of the formula -CH₂-CH₂- or -CH=CH-
and
R
- represents a group of the formula 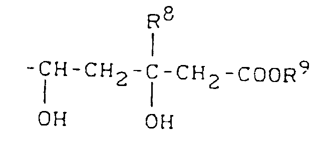 or 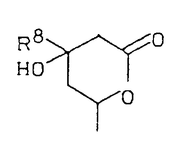
in which
R⁸
- denotes hydrogen
and
R⁹
- denotes hydrogen or straight-chain or branched alkyl having up to 8 carbon atoms or benzyl, or
- denotes phenyl or a cation,
and their salts.

10. Process for the preparation of ketones according to Claim 9
characterized in that aldehydes of the general formula (IX) 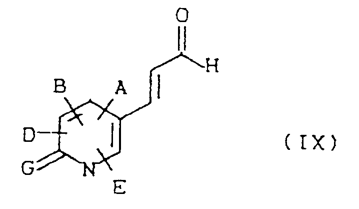 in which
A, B, D, E and G have the abovementioned meaning,
are reacted in inert solvents with acetoacetic esters of the general formula (X) 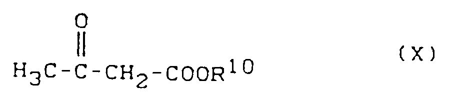 in which
R¹⁰ has the abovementioned meaning,
in the presence of bases.

## Claims (Claims for the following Contracting State(s): ES)

1. Process for the preparation of substituted 2-pyridones and pyrid-2-thiones of the general formulae (Ia) and (Ib) 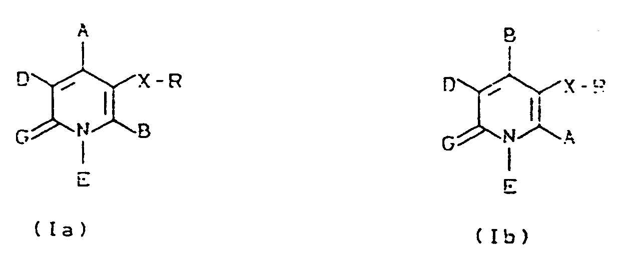 in which
A
- represents oxiranyl, thienyl, furyl, pyridyl, pyrimidyl, pyrazinyl, indolyl, quinolyl, isoquinolyl, benzothiazolyl or benzimidazolyl, each of which is optionally monosubstituted to tetrasubstituted by identical or different substituents from the series comprising fluorine, chlorine, bromine, trifluoromethyl, trifluoromethylthio, trifluoromethoxy, straight-chain or branched alkyl, alkylthio, alkylsulphonyl, alkoxy or alkoxycarbonyl each having up to 6 carbon atoms, phenyl, phenylthio, phenylsulphonyl or by a group of the formula -NR¹R²,
in which
R¹ and R² are identical or different and
- denote hydrogen, phenyl, phenylsulphonyl, straight-chain or branched alkyl or alkylsulphonyl having up to 6 carbon atoms, benzyl or benzylsulphonyl, or
- denote a group of the formula -COR³,
in which
R³
- denotes straight-chain or branched alkyl or alkoxy having up to 6 carbon atoms or phenyl,
or
R¹ and R², together with the nitrogen atom, form a 5- to 7-membered ring which may be substituted by straight-chain or branched alkyl having up to 6 carbon atoms,
- represents phenyl or naphthyl, each of which is optionally monosubstituted to tetrasubstituted by identical or different substituents from the series comprising straight-chain or branched alkyl, alkylthio, alkylsulphonyl, alkoxy or alkoxycarbonyl each having up to 8 carbon atoms, which may in turn be substituted by hydroxyl, alkoxy having up to 4 carbon atoms, phenyl or by a group of the formula -NR¹R²,
or by phenyl, phenyloxy, phenylthio, phenylsulphonyl, fluorine, chlorine, bromine, nitro, cyano, trifluoromethyl, trifluoromethoxy, trifluoromethylthio, benzyloxy or by a group of the formula -NR¹R²,
B
- represents cyclopropyl, cyclobutyl, cyclopentyl or cyclohexyl,
- represents straight-chain or branched alkyl having up to 10 carbon atoms, which is optionally substituted by fluorine, chlorine, bromine, cyano, azido, trifluoromethyl, trifluoromethoxy, trifluoromethylthio, trifluoromethylsulphonyl, alkoxy having up to 8 carbon atoms or by phenyl, phenyloxy, or phenylthio, thienyl, furyl, pyridyl, pyrimidyl or quinolyl which may in turn be monosubstituted or disubstituted by identical or different substituents from the series comprising fluorine, chlorine, bromine, cyano, trifluoromethyl, trifluoromethoxy, straight-chain or branched alkyl, alkoxy, alkylthio or alkylsulphonyl each having up to 6 carbon atoms, or by a group of the formula -NR¹R² or -COR³,
- represents phenyl which is optionally substituted by substituents from the series comprising fluorine, chlorine, bromine, cyano, nitro, trifluoromethyl, straight-chain or branched alkyl, alkoxy or alkoxycarbonyl each having up to 6 carbon atoms or amino,
D and E are identical or different and have the above mentioned meaning of A and are identical or different to this, or
- represent hydrogen, nitro or cyano,
- represent cyclopropyl, cyclobutyl, cyclopentyl or cyclohexyl,
- represent straight-chain or branched alkyl or alkenyl each having up to 10 carbon atoms or nitrilo which is optionally substituted by fluorine, chlorine, bromine, azido, 2,5-dioxo-tetrahydropyrryl, phenyl, pyrimidyl, pyrryl, pyrrolidinyl, morpholino or morpholino-N-oxide, or by a group of the formula NR¹R², -OR⁴, -COR⁵ or S(O)ₙ-R⁶,
in which
R¹ and R² have the abovementioned meaning,
R⁴
- denotes hydrogen or
- denotes straight-chain or branched alkyl having up to 8 carbon atoms, which is optionally substituted by hydroxyl, trialkylsilyl having up to 8 carbon atoms in the alkyl moiety, fluorine, chlorine, bromine or by phenyl which may in turn be substituted by fluorine, chlorine, bromine, cyano, nitro, hydroxyl, straight-chain or branched alkyl, alkoxy or alkoxycarbonyl each having up to 6 carbon atoms or amino,
- denotes trialkylsilyl having up to 8 carbon atoms, tetrahydropyranyl or 2,5-dioxo-tetrahydropyrryl,
- denotes cyclopropyl, cyclobutyl, cyclopentyl, cyclohexyl or phenyl which may in turn be substituted by fluorine, chlorine, bromine, cyano, nitro or amino, or
- denotes a group of the formula -COR⁷,
in which
R⁷
- denotes straight-chain or branched alkyl having up to 6 carbon atoms, phenyl or a group of the formula -NR¹R²,
R⁵
- denotes hydrogen, straight-chain or branched alkyl having up to 8 carbon atoms, which is optionally substituted by hydroxyl, phenyl, fluorine, chlorine, bromine or cyano,
- denotes phenyl, naphthyl, pyrryl, pyrimidyl, pyridyl, pyrrolidinyl or morpholino which may in turn be substituted by fluorine, chlorine, bromine, amino, hydroxyl, nitro or cyano, or
- denotes a group of the formula -NR¹R² or -OR⁴,
n
- denotes a number 0 or 2,
R⁶
- denotes straight-chain or branched alkyl having up to 8 carbon atoms, which may be substituted by fluorine, chlorine, bromine, hydroxyl, phenyl or by a group of the formula -NR¹R²,
- denotes phenyl which may be substituted by fluorine, chlorine, bromine, hydroxyl, cyano, nitro or amino, or
- denotes a group of the formula -NR¹R² if n represents the number 2,
or
D and E are identical or different and
- represent a group of the formula -NR¹R², -OR⁴ or -COR⁵,
in which
R¹, R², R⁴ and R⁵ have the abovementioned meaning,
or
D and E together with B form a 5- to 7-membered saturated or unsaturated ring which is optionally substituted by straight-chain or branched alkyl having up to 6 carbon atoms or phenyl,
G
- represents an oxygen or a sulphur atom,
X
- represents a group of the formula -CH₂-CH₂- or -CH=CH-
and
R
- represents a group of the formula 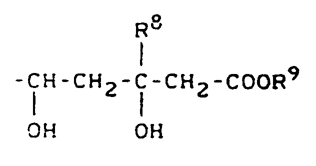 or 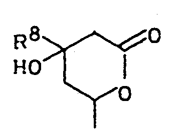
in which
R⁸
- denotes hydrogen
and
R⁹
- denotes hydrogen or straight-chain or branched alkyl having up to 8 carbon atoms or benzyl, or
- denotes phenyl or a cation,
or
D
- represents the group of the formula -X-R,
in which
X and R have the abovementioned meaning,
and their salts characterized in that,
ketones of the general formula (VIII) 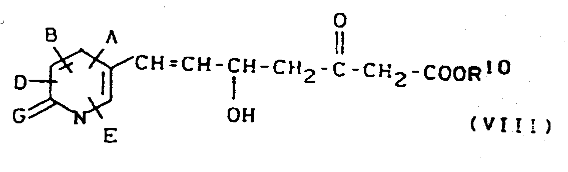 in which
A, B, D, K and G have the abovementioned meaning,
and
R¹⁰ - represents alkyl,
are reduced,
in the case of the preparation of the acids the esters are hydrolyzed,
in the case of the preparation of the lactones the carboxylic acids are cyclized,
in the case of the preparation of the salts either the esters or the lactones are hydrolyzed,
in the case of the preparation of the ethylene compounds (X = -CH₂-CH₂-) the ethene compounds (X= -CH=CH-) are hydrogenated by customary methods,
and, if appropriate, isomers are separated.

2. Process for the preparation of ketones of the formula 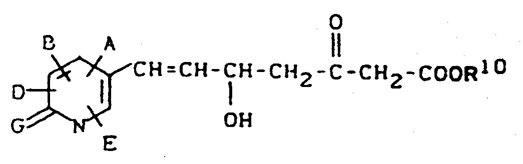 in which
A, B, D, E, G and R¹⁰ have the meaning given in Claim 1,
characterized in that aldehydes of the general formula (IX) 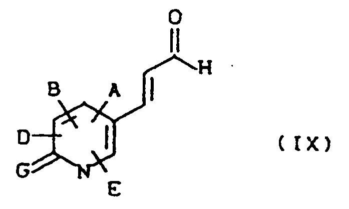 in which
A, B, D, E and G have the abovementioned meaning,
are reacted in inert solvents with acetoacetic esters of the general formula (X) 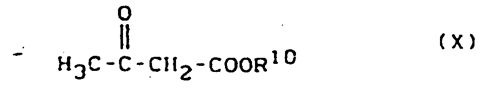 which
R¹⁰ has the abovementioned meaning,
in the presence of bases.

## Revendications (Revendications pour l'(les) Etat(s) contractant(s) suivant(s): AT, BE, CH, DE, FR, GB, GR, IT, LI, LU, NL, SE)

1. 2-pyridones et pyrid-2-thiones de formules générales (Ia) et (Ib) 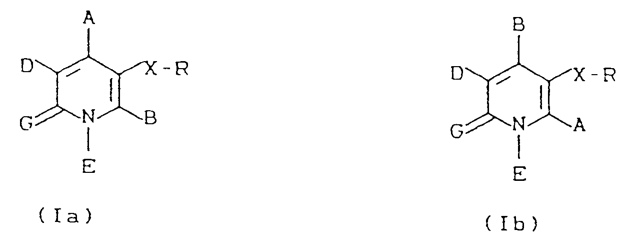 dans lesquelles
A
- est un groupe oxiranyle, thiényle, furyle, pyridyle, pyrimidyle, pyrazinyle, indolyle, quinolyle, isoquinolyle, benzothiazolyle ou benzimidazolyle qui porte le cas échéant jusqu'à 4 substituants, identiques ou différents, fluoro, chloro, bromo, trifluorométhyle, trifluorométhylthio, trifluorométhoxy, alkyle, alkylthio, alkylsulfonyle, alkoxy ou alkoxycarbonyle à chaîne droite ou ramifiée ayant chacun jusqu'à 6 atomes de carbone, phényle, phénylthio, phénylsulfonyle ou groupe de formule -NR¹R²,
où
R¹ et R² sont identiques ou différents et représentent
- l'hydrogène, un groupe phényle, phénylsulfonyle, un groupe alkyle ou alkylsulfonyle à chaîne droite ou ramifiée ayant jusqu'à 6 atomes de carbone, un groupe benzyle ou benzylsulfonyle, ou bien
- un groupe de formule -COR³,
dans laquelle
R³
- est un groupe alkyle ou alkoxy linéaire ou ramifié ayant jusqu'à 6 atomes de carbone ou un groupe phényle,
ou bien
R¹ et R² forment, conjointement avec l'atome d'azote, un noyau pentagonal à heptagonal qui peut être substitué par un radical alkyle linéaire ou ramifié ayant jusqu'à 6 atomes de carbone,
- un groupe phényle ou naphtyle qui porte le cas échéant jusqu'à 4 substituants identiques ou différents, alkyle, alkylthio, alkylsulfonyle, alkoxy ou alkoxycarbonyle linéaires ou ramifiés ayant chacun jusqu'à 8 atomes de carbone et pouvant être substitués quant à eux par un radical hydroxy, alkoxy ayant jusqu'à 4 atomes de carbone, phényle ou par un groupe de formule -NR¹R²,
ou phényle, phényloxy, phénylthio, phénylsulfonyle, fluoro, chloro, bromo, nitro, cyano, trifluorométhyle, trifluorométhoxy, trifluorométhylthio, benzyloxy ou groupe de formule -NR¹R²,
B
- est un groupe cyclopropyle, cyclobutyle, cyclopentyle ou cyclohexyle,
- un groupe alkyle à chaîne droite ou ramifiée ayant jusqu'à 10 atomes de carbone, qui est substitué le cas échéant par du fluor, du chlore, du brome, un radical cyano, azido, trifluorométhyle, trifluorométhoxy, trifluorométhylthio, trifluorométhylsulfonyle, alkoxy ayant jusqu'à 8 atomes de carbone ou par un radical phényle, phényloxy ou phénylthio, thiényle, furyle, pyridyle, pyrimidyle ou quinolyle, qui peut porter quant à lui jusqu'à 2 substituants identiques ou différents, fluoro, chloro, bromo, cyano, trifluorométhyle, trifluorométhoxy, alkyle, alkoxy, alkylthio ou alkylsulfonyle à chaîne droite ou ramifiée avec chacun jusqu'à 6 atomes de carbone, ou bien par un groupe de formule -NR¹R² ou -COR³,
- un groupe phényle qui porte le cas échéant jusqu'à 2 substituants, identiques ou différents, fluoro, chloro, bromo, cyano, nitro, trifluorométhyle, alkyle, alkoxy ou alkoxycarbonyle linéaire ou ramifié avec chacun jusqu'à 6 atomes de carbone, ou amino,
D et E sont identiques ou différents et ont la définition indiquée ci-dessus pour A et sont identiques à A ou en sont différents, ou représentent
- l'hydrogène, un groupe nitro ou cyano,
- un groupe cyclopropyle, cyclobutyle, cyclopentyle ou cyclohexyle,
- un groupe alkyle ou alcényle linéaire ou ramifié avec chacun jusqu'à 10 atomes de carbone ou un groupe imino, qui est substitué le cas échéant par du fluor, du chlore, du brome, un radical azido, 2,5-dioxotétrahydropyrryle, phényle, pyrimidyle, pyrryle, pyrrolidinyle, morpholino ou morpholino-N-oxyde, ou sont substitués par un groupe de formule NR¹R², -OR⁴, -COR⁵ ou -S(O)ₙ-R⁶,
où
R¹ et R² ont la définition indiquée ci-dessus,
R⁴
- représente l'hydrogène ou
- un groupe alkyle à chaîne droite ou ramifiée ayant jusqu'à 8 atomes de carbone, qui est substitué le cas échéant par un radical hydroxy, trialkylsilyle ayant jusqu'à 8 atomes de carbone dans l'ensemble de la partie alkyle, un radical fluoro, chloro, bromo ou un radical phényle qui peut être substitué quant à lui par du fluor, du chlore, du brome, un radical cyano, nitro, hydroxy, alkyle, alkoxy ou alkoxycarbonyle à chaîne droite ou ramifiée ayant chacun jusqu'à 6 atomes de carbone ou amino,
- un groupe trialkylsilyle ayant jusqu'à 8 atomes de carbone dans le total de la partie alkyle, tétrahydropyrannyle ou 2,5-dioxo-tétrahydropyrryle,
- un groupe cyclopropyle, cyclobutyle, cyclopentyle, cyclohexyle ou phényle, qui peut être substitué de son côté par du fluor, du chlore, du brome, un radical cyano, nitro ou amino, ou bien
- un groupe de formule -COR⁷,
dans laquelle
R⁷
- est un groupe alkyle linéaire ou ramifié ayant jusqu'à 6 atomes de carbone, un groupe phényle ou un groupe de formule -NR¹R²,
R⁵
- représente l'hydrogène, un groupe alkyle linéaire ou ramifié ayant jusqu'à 8 atomes de carbone, qui est substitué le cas échéant par un radical hydroxy, phényle, fluoro, chloro, bromo ou cyano,
- un groupe phényle, naphtyle, pyrryle, pyrimidyle, pyridyle, pyrrolidinyle ou morpholino qui peut être substitué quant à lui par du fluor, du chlore, du brome, un radical amino, hydroxy, nitro ou cyano, ou bien
- un groupe de formule -NR¹R² ou -OR⁴
n
- représente le nombre 0 ou 2,
R⁶
- est un groupe alkyle à chaîne droite ou ramifiée ayant jusqu'à 8 atomes de carbone, qui peut être substitué par du fluor, du chlore, du brome, un radical hydroxy, phényle ou un groupe de formule -NR¹R²,
- un groupe phényle qui peut être substitué par du fluor, du chlore, du brome, un radical hydroxy, cyano, nitro ou amino, ou
- un groupe de formule -NR¹R² lorsque n représente le nombre 2,
ou bien
D et E sont identiques ou différents et représentent
- un groupe de formules -NR¹R², -OR⁴ ou -COR⁵,
dans laquelle
R¹, R², R⁴ et R⁵ ont la définition indiquée ci-dessus,
D ou E forment conjointement avec B un noyau pentagonal à heptagonal saturé ou non saturé, qui est substitué le cas échéant par un radical alkyle linéaire ou ramifié ayant jusqu'à 6 atomes de carbone ou un radical phényle,
G
- est un atome d'oxygène ou un atome de soufre,
X
- est un groupe de formule -CH₂-CH₂- ou -CH=CH-
et
R
- est un groupe de formule 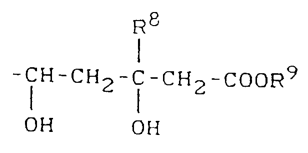 ou 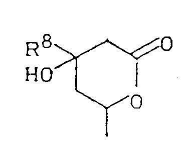
dans laquelle
R⁸
- représente l'hydrogène
R⁹
- est l'hydrogène ou un groupe alkyle linéaire ou ramifié, ayant jusqu'à 8 atomes de carbone ou un groupe benzyle, ou bien un groupe phényle ou un cation, ou bien
D
- représente le groupe de formule -X-R
dans laquelle
X et R ont la définition indiquée ci-dessus,
et leurs sels.

2. 2-pyridones et pyrid-2-thiones suivant la revendication 1, dans lesquelles
A
- est un groupe oxiranyle, pyridyle, pyrimidyle, quinolyle ou isoquinolyle qui porte le cas échéant jusqu'à 3 substituants, identiques ou différents, fluoro, chloro, trifluorométhyle, alkyle, alkylthio, alkylsulfonyle, alkoxy ou alkoxycarbonyle linéaire ou ramifié avec chacun jusqu'à 4 atomes de carbone, phényle, phénylthio, phénylsulfonyle ou groupe de formule -NR¹R²,
où
R¹ et R² sont identiques ou différents et représentent
- l'hydrogène, un groupe phényle, phénylsulfonyle, alkyle ou alkylsulfonyle linéaire ou ramifié ayant jusqu'à 4 atomes de carbone, benzyle ou benzylsulfonyle,
- un groupe de formule -COR³,
dans laquelle
R³
- est un groupe alkyle ou alkoxy linéaire ou ramifié ayant jusqu'à 4 atomes de carbone ou phényle,
ou bien
R¹ et R² forment, conjointement avec l'atome d'azote, un noyau pentagonal à heptagonal qui peut être substitué par un groupe alkyle linéaire ou ramifié ayant jusqu'à 4 atomes de carbone,
- un groupe phényle qui porte le cas échéant 1 à 3 substituants, identiques ou différents, alkyle, alkylthio, alkylsulfonyle, alkoxy ou alkoxycarbonyle linéaires ou ramifiés ayant chacun jusqu'à 6 atomes de carbone, qui peuvent être substitués quant à eux par un radical hydroxy, méthoxy, éthoxy, propoxy, phényle ou par un groupe de formule -NR¹R²,
ou phényle, phényloxy, fluoro, chloro, nitro, cyano, trifluorométhyle, benzyloxy ou groupe de formule -NR¹R²,
où
R¹ et R² ont la définition indiquée ci-dessus,
B
- représente
un groupe cyclopropyle, cyclobutyle, cyclopentyle ou cyclohexyle,
- un groupe alkyle à chaîne droite ou ramifiée ayant jusqu'à 8 atomes de carbone, qui est substitué le cas échéant par des radicaux fluoro, chloro, cyano, azido, alkoxy ayant jusqu'à 6 atomes de carbone, phényle ou phénoxy, qui sont substitués quant à eux par du fluor, du chlore, un radical cyano, alkyle ou alkoxy linéaire ou ramifié ayant jusqu'à 4 atomes de carbone ou par un groupe de formule -NR¹R² ou -COR³,
- un groupe phényle qui est substitué par du fluor, du chlore, un radical nitro, alkyle, alkoxy ou alkoxycarbonyle linéaire ou ramifié avec chacun jusqu'à 4 atomes de carbone, ou amino
D et E sont identiques ou différents et ont la définition donnée ci-dessus pour A et sont identiques à A ou en sont différents, ou représentent
- de l'hydrogène, un groupe nitro ou cyano,
- un groupe cyclopropyle, cyclopentyle ou cyclohexyle,
- un groupe alkyle ou alcényle linéaire ou ramifié avec chacun jusqu'à 8 atomes de carbone ou imino qui porte le cas échéant un substituant fluoro, chloro, azido, 2,5-dioxo-tétrahydropyrryle, phényle, pyrrolidinyle, morpholino ou morpholino-N-oxyde, ou qui sont substitués par un groupe de formules -NR¹R², -OR⁴, -COR⁵ ou -S(O)ₙ-R⁶,
où
R¹ et R² ont la définition indiquée ci-dessus,
R⁴
- représente de l'hydrogène ou
- un groupe alkyle à chaîne droite ou ramifiée ayant jusqu'à 6 atomes de carbone, qui est substitué le cas échéant par un radical hydroxy, diméthyl-tertio-butylsilyle, fluoro, chloro ou par un radical phényle qui peut être substitué quant à lui par du fluor, du chlore, un radical hydroxy ou amino,
- un groupe trialkylsilyle ayant jusqu'à 6 atomes de carbone dans l'ensemble de la partie alkyle, tétrahydropyrannyle ou 2,5-dioxo-tétra-hydropyrryle,
- un groupe cyclopropyle, cyclopentyle, cyclohexyle ou phényle, ou
- un groupe de formule -COR⁷,
dans laquelle
R⁷
- est un groupe alkyle à chaîne droite ou ramifiée ayant jusqu'à 4 atomes de carbone, un groupe phényle ou un groupe de formule -NR¹R²,
R⁵
- représente
l'hydrogène, un groupe alkyle linéaire ou ramifié ayant jusqu'à 6 atomes de carbone, qui est substitué le cas échéant par un radical hydroxy, phényle, fluoro ou chloro,
- un groupe phényle, pyrryle, pyrrolidinyle ou morpholino, ou
- un groupe de formule -NR¹R² ou -OR⁴,
n
- représente le nombre 0 ou 2,
R⁶
- représente
- un groupe alkyle linéaire ou ramifié ayant jusqu'à 6 atomes de carbone, qui peut être substitué par du fluor, du chlore, un radical hydroxy, phényle ou par un groupe de formule -NR¹R²,
- un groupe phényle qui peut être substitué par du fluor, du chlore, un radical hydroxy, cyano, nitro ou amino,
- un groupe de formule -NR¹R², lorsque n représente le nombre 2,
ou bien
D et E sont identiques ou différents et représentent
- un groupe de formule -NR¹R², -OR⁴ ou -COR⁵,
dans laquelle
R¹, R², R⁴ et R⁵ ont la définition indiquée ci-dessus,
ou bien
D et E forment conjointement avec B un noyau saturé ou non saturé pentagonal à heptagonal qui est substitué le cas échéant par un radical méthyle, éthyle, propyle, isopropyle, butyle, tertio-butyle ou phényle,
G
- représente un atome d'oxygène ou de soufre,
X
- est un groupe -CH=CH-
et
R
- est un groupe de formule 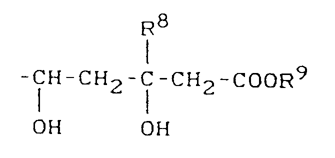 ou 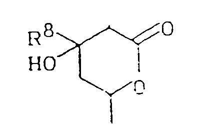
dans laquelle
R⁸
- représente l'hydrogène,
et
R⁹
- représente l'hydrogène, un groupe méthyle, éthyle, propyle, isopropyle, butyle, isobutyle, tertio-butyle ou benzyle, ou bien
- un ion sodium, potassium, calcium, magnésium ou ammonium
ou
D
- représente un groupe de formule -X-R
dans laquelle
X et R ont la définition indiquée ci-dessus,
ou leurs sels.

3. Procédé de production de 2-pyridones et de pyrid-2-thiones substituées suivant les revendications 1 et 2, caractérisé en ce qu'on réduit des cétones de formule générale (VIII) 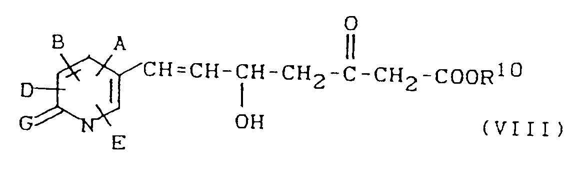 dans laquelle
A, B, D, E et G ont la définition indiquée ci-dessus, et
R¹⁰ - est un groupe alkyle,
dans le cas de la production des acides, on saponifie les esters,
dans le cas de la production des lactones, on cyclise les acides carboxyliques,
dans le cas de la production des sels, on saponifie les esters ou les lactones,
dans le cas de la production des composés éthyléniques (X = -CH₂-CH₂-), on hydrogène les composés éthéniques (X = -CH=CH-) par des procédés classiques,
et on sépare éventuellement les isomères.

4. 2-pyridone et pyrid-2-thiones suivant la revendication 1, destinées à combattre des maladies.

5. Médicaments contenant au moins une 2-pyridone ou une pyrid-2-thione suivant la revendication 1.

6. Procédé de préparation d'un médicament suivant la revendication 5, caractérisé en ce qu'on met sous une forme d'administration appropriée des 2-pyridones ou des pyrid-2-thiones suivant la revendication 1, le cas échéant à l'aide de substances auxiliaires et de supports classiques.

7. Utilisation des 2-pyridones et pyrid-2-thiones suivant la revendication 1 pour la préparation de médicaments.

8. Utilisation des 2-pyridones et pyrid-2-thiones suivant la revendication 1 pour la préparation de médicaments destinés au traitement de l'hyperlipoprotéinémie, de la lipoprotéinémie ou de l'artériosclérose.

9. Cétones de formule générale (VIII) 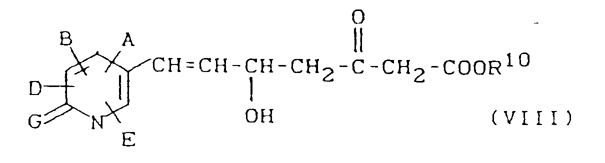 dans laquelle
A
- représente un groupe oxiranyle, thiényle, furyle, pyridyle, pyrimidyle, pyrazinyle, indolyle, quinolyle, isoquinolyle, benzothiazolyle ou benzimidazolyle qui portent le cas échéant jusqu'à 4 substituants, identiques ou différents, fluoro, chloro, bromo, trifluorométhyle, trifluorométhylthio, trifluorométhoxy, alkyle, alkylthio, alkylsulfonyle, alkoxy ou alkoxycarbonyle linéaires ou ramifiés ayant chacun jusqu'à 6 atomes de carbone, phényle, phénylthio, phénylsulfonyle ou groupe de formule -NR¹R²,
dans laquelle
R¹ et R² sont identiques ou différents et représentent
- l'hydrogène, un groupe phényle, phénylsulfonyle, alkyle ou alkylsulfonyle à chaîne droite ou ramifiée ayant jusqu'à 6 atomes de carbone, benzyle ou benzylsulfonyle, ou bien
- un groupe de formule -COR³,
dans laquelle
R³
- est un groupe alkyle ou alkoxy linéaire ou ramifié ayant jusqu'à 6 atomes de carbone ou phényle,
ou bien
R¹ et R² forment, conjointement avec l'atome d'azote, un noyau pentagonal à heptagonal qui peut être substitué par un radical alkyle linéaire ou ramifié ayant jusqu'à 6 atomes de carbone,
- un groupe phényle ou naphtyle qui porte le cas échéant jusqu'à 4 substituants, identiques ou différents, alkyle, alkylthio, alkylsulfonyle, alkoxy ou alkoxycarbonyle, linéaires ou ramifiés avec chacun jusqu'à 8 atomes de carbone, qui peuvent être substitués quant à eux par un radical hydroxy, alkoxy ayant jusqu'à 4 atomes de carbone, phényle ou par un groupe de formule -NR¹R²,
ou phényle, phényloxy, phénylthio, phénylsulfonyle, fluoro, chloro, bromo, nitro, cyano, trifluorométhyle, trifluorométhoxy, trifluorométhylthio, benzyloxy ou groupe de formule -NR¹R²,
B
- représente
- un groupe cyclopropyle, cyclobutyle, cyclopentyle ou cyclohexyle,
- un groupe alkyle linéaire ou ramifié ayant jusqu'à 10 atomes de carbone, qui est substitué le cas échéant par du fluor, du chlore, du brome, un radical cyano, azido, trifluorométhyle, trifluorométhoxy, trifluorométhylthio, trifluorométhylsulfonyle, alkoxy ayant jusqu'à 8 atomes de carbone, ou phényle, phényloxy, phénylthio, thiényle, furyle, pyridyle, pyrimidyle ou quinolyle qui peut porter quant à lui jusqu'à 2 substituants, identiques ou différents, fluoro, chloro, bromo, cyano, trifluorométhyle, trifluorométhoxy, alkyle, alkoxy, alkylthio ou alkylsulfonyle linéaire ou ramifié avec chacun jusqu'à 6 atomes de carbone, ou par un groupe de formule -NR¹R² ou -COR³,
- un groupe phényle qui porte le cas échéant jusqu'à 3 substituants, identiques ou différents, fluoro, chloro, bromo, cyano, nitro, trifluorométhyle, alkyle, alkoxy ou alkoxycarbonyle linéaires ou ramifiés ayant chacun jusqu'à 6 atomes de carbone ou amino,
D et E sont identiques ou différents et ont la définition indiquée ci-dessus pour A et sont identiques à A ou en sont différents, ou représentent
- l'hydrogène, un groupe nitro ou cyano,
- un groupe cyclopropyle, cyclobutyle, cyclopentyle ou cyclohexyle,
- un groupe alkyle ou alcényle linéaire ou ramifié avec chacun jusqu'à 10 atomes de carbone ou imino, portant le cas échéant un substituant fluoro, chloro, bromo, azido, 2,5-dioxotétrahydropyrryle, phényle, pyrimidyle, pyrryle, pyrrolidinyle, morpholino ou morpholino-N-oxyde, ou sont substitués par un groupe de formule NR¹R², -OR⁴, -COR⁵ ou -S(O)ₙ-R⁶,
où
R¹ et R² ont la définition indiquée ci-dessus,
R⁴
- représente
- l'hydrogène ou
- un groupe alkyle à chaîne droite ou ramifiée ayant jusqu'à 8 atomes de carbone, qui est substitué le cas échéant par un radical hydroxy, trialkylsilyle avec jusqu'à 8 atomes de carbone dans l'ensemble de la partie alkyle, fluoro, chloro, bromo ou phényle, qui peut être substitué quant à lui par un radical fluoro, chloro, bromo, cyano, nitro, hydroxy, alkyle, alkoxy ou alkoxycarbonyle linéaire ou ramifié avec chacun jusqu'à 6 atomes de carbone, ou amino
- un groupe trialkylsilyle avec jusqu'à 8 atomes de carbone dans le total de la partie alkyle, tétrahydropyrannyle ou 2,5-dioxo-tétrahydropyrryle,
- un groupe cyclopropyle, cyclobutyle, cyclopentyle, cyclohexyle ou phényle qui peut être substitué quant à lui par un radical fluoro, chloro, bromo, cyano, nitro ou amino, ou bien
- un groupe de formule -COR⁷,
dans laquelle
R⁷
- est un groupe alkyle à chaîne droite ou ramifiée ayant jusqu'à 6 atomes de carbone, phényle ou un groupe de formule -NR¹R²,
R⁵
- représente l'hydrogène, un groupe alkyle linéaire ou ramifié ayant jusqu'à 8 atomes de carbone, qui est substitué le cas échéant par un radical hydroxy, phényle, fluoro, chloro, bromo ou cyano,
- un groupe phényle, naphtyle, pyrryle, pyrimidyle, pyridyle, pyrrolidinyle ou morpholino qui peut être substitué quant à lui par un radical fluoro, chloro, bromo, amino, hydroxy, nitro ou cyano, ou bien
- un groupe de formule -NR¹R² ou -OR⁴,
n
- est le nombre 0 ou 2,
R⁶
- est un groupe alkyle linéaire ou ramifié avec jusqu'à 8 atomes de carbone, qui peut être substitué par un radical fluoro, chloro, bromo, hydroxy, phényle ou par un groupe de formule -NR¹R²,
- un groupe phényle qui peut être substitué par un radical fluoro, chloro, bromo, hydroxy, cyano, nitro ou amino, ou bien
- un groupe de formule -NR¹R², lorsque n représente le nombre 2,
ou bien
D et E sont identiques ou différents et représentent
- un groupe de formule -NR¹R², -OR⁴ ou -COR⁵,
où
R¹, R², R⁴ et R⁵ ont la définition indiquée ci-dessus,
D ou E forment conjointement avec B un noyau pentagonal à heptagonal saturé ou non saturé, qui est substitué le cas échéant par un radical alkyle linéaire ou ramifié ayant jusqu'à 6 atomes de carbone ou phényle,
G
- représente un atome d'oxygène ou un atome de soufre,
ou bien
D
- représente le groupe de formule -X-R-,
X
- est un groupe de formule -CH₂-CH₂ ou -CH=CH-
et
R
- est un groupe de formule 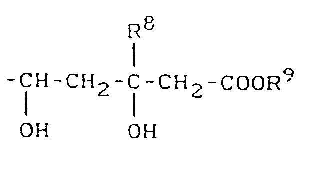 ou 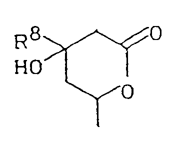
dans laquelle
R⁸
- représente l'hydrogène
R⁹
- représente l'hydrogène ou un groupe alkyle linéaire ou ramifié ayant jusqu'à 8 atomes de carbone, ou benzyle, ou bien
- un groupe phényle ou un cation,
et leurs sels.

10. Procédé de production de cétones suivant la revendication 9, caractérisé en ce qu'on fait réagir en présence de bases des aldéhydes de formule générale (IX) 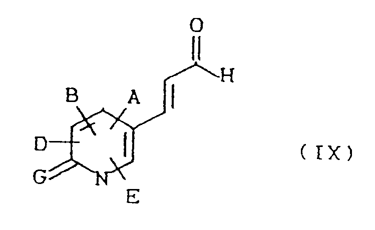 dans laquelle
A, B, D, E et G ont la définition indiquée ci-dessus,
dans des solvants inertes, avec un ester acétylacétique de formule générale (X) 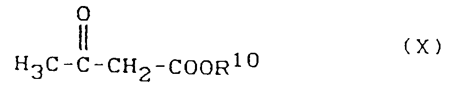 dans laquelle
R¹⁰ a la définition indiquée ci-dessus.

## Revendications (Revendications pour l'(les) Etat(s) contractant(s) suivant(s): ES)

1. Procédé de production de 2-pyridones et de pyrid-2-thiones de formules générales (Ia) et (Ib) 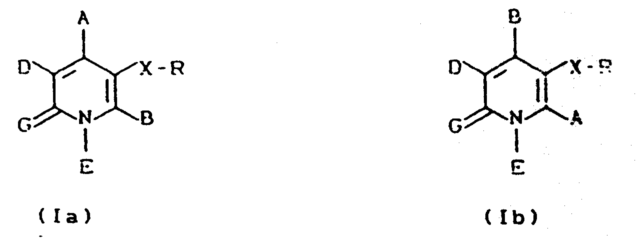 dans lesquelles
A
- est un groupe oxiranyle, thiényle, furyle, pyridyle, pyrimidyle, pyrazinyle, indolyle, quinolyle, isoquinolyle, benzothiazolyle ou benzimidazolyle qui porte le cas échéant jusqu'à 4 substituants, identiques ou différents, fluoro, chloro, bromo, trifluorométhyle, trifluorométhylthio, trifluorométhoxy, alkyle, alkylthio, alkylsulfonyle, alkoxy ou alkoxycarbonyle à chaîne droite ou ramifiée ayant chacun jusgu'à 6 atomes de carbone, phényle, phénylthio, phénylsulfonyle ou groupe de formule -NR¹R²,
où
R¹ et R² sont identiques ou différents et représentent
- l'hydrogène, un groupe phényle, phénylsulfonyle, un groupe alkyle ou alkylsulfonyle à chaîne droite ou ramifiée ayant jusqu'à 6 atomes de carbone, un groupe benzyle ou benzylsulfonyle, ou bien
- un groupe de formule -COR³,
dans laquelle
R³
- est un groupe alkyle ou alkoxy linéaire ou ramifié ayant jusqu'à 6 atomes de carbone ou un groupe phényle,
ou bien
R¹ et R² forment, conjointement avec l'atome d'azote, un noyau pentagonal à heptagonal qui peut être substitué par un radical alkyle linéaire ou ramifié ayant jusqu'à 6 atomes de carbone,
- un groupe phényle ou naphtyle qui porte le cas échéant jusqu'à 4 substituants identiques ou différents, alkyle, alkylthio, alkylsulfonyle, alkoxy ou alkoxycarbonyle linéaires ou ramifiés ayant chacun jusqu'à 8 atomes de carbone et pouvant être substitués quant à eux par un radical hydroxy, alkoxy ayant jusqu'à 4 atomes de carbone, phényle ou par un groupe de formule -NR¹R²,
ou phényle, phényloxy, phénylthio, phénylsulfonyle, fluoro, chloro, bromo, nitro, cyano, trifluorométhyle, trifluorométhoxy, trifluorométhylthio, benzyloxy ou groupe de formule -NR¹R²,
B
- est un groupe cyclopropyle, cyclobutyle, cyclopentyle ou cyclohexyle,
- un groupe alkyle à chaîne droite ou ramifiée ayant jusqu'à 10 atomes de carbone, qui est substitué le cas échéant par du fluor, du chlore, du brome, un radical cyano, azido, trifluorométhyle, trifluorométhoxy, trifluorométhylthio, trifluorométhylsulfonyle, alkoxy ayant jusqu'à 8 atomes de carbone ou par un radical phényle, phényloxy ou phénylthio, thiényle, furyle, pyridyle, pyrimidyle ou quinolyle, qui peut porter quant à lui jusqu'à 2 substituants identiques ou différents, fluoro, chloro, bromo, cyano, trifluorométhyle, trifluorométhoxy, alkyle, alkoxy, alkylthio ou alkylsulfonyle à chaîne droite ou ramifiée avec chacun jusqu'à 6 atomes de carbone, ou bien
- par un groupe de formule -NR¹R² ou -COR³,
- un groupe phényle qui porte le cas échéant jusqu'à 2 substituants, identiques ou différents, fluoro, chloro, bromo, cyano, nitro, trifluorométhyle, alkyle, alkoxy ou alkoxycarbonyle linéaire ou ramifié avec chacun jusqu'à 6 atomes de carbone, ou amino,
D et E sont identiques ou différents et ont la définition indiquée ci-dessus pour A et sont identiques à A ou en sont différents, ou représentent
- l'hydrogène, un groupe nitro ou cyano,
- un groupe cyclopropyle, cyclobutyle, cyclopentyle ou cyclohexyle,
- un groupe alkyle ou alcényle linéaire ou ramifié avec chacun jusqu'à 10 atomes de carbone ou un groupe imino, qui est substitué le cas échéant par du fluor, du chlore, du brome, un radical azido, 2,5-dioxotétrahydropyrryle, phényle, pyrimidyle, pyrryle, pyrrolidinyle, morpholino ou morpholino-N-oxyde, ou sont substitués par un groupe de formule NR¹R², -OR⁴, -COR⁵ ou -S(O)ₙ-R⁶,
où
R¹ et R² ont la définition indiquée ci-dessus,
R⁴
- représente l'hydrogène ou
- un groupe alkyle à chaîne droite ou ramifiée ayant jusqu'à 8 atomes de carbone, qui est substitué le cas échéant par un radical hydroxy, trialkylsilyle ayant jusqu'à 8 atomes de carbone dans l'ensemble de la partie alkyle, un radical fluoro, chloro, bromo ou un radical phényle qui peut être substitué quant à lui par du fluor, du chlore, du brome, un radical cyano, nitro, hydroxy, alkyle, alkoxy ou alkoxycarbonyle à chaîne droite ou ramifiée ayant chacun jusqu'à 6 atomes de carbone ou amino,
- un groupe trialkylsilyle ayant jusqu'à 8 atomes de carbone dans le total de la partie alkyle, tétrahydropyrannyle ou 2,5-dioxo-tétrahydropyrryle,
- un groupe cyclopropyle, cyclobutyle, cyclopentyle, cyclohexyle ou phényle, qui peut être substitué de son côté par du fluor, du chlore, du brome, un radical cyano, nitro ou amino, ou bien
- un groupe de formule -COR⁷,
dans laquelle
R⁷
- est un groupe alkyle linéaire ou ramifié ayant jusqu'à 6 atomes de carbone, un groupe phényle ou un groupe de formule -NR¹R²,
R⁵
- représente l'hydrogène, un groupe alkyle linéaire ou ramifié ayant jusqu'à 8 atomes de carbone, qui est substitué le cas échéant par un radical hydroxy, phényle, fluoro, chloro, bromo ou cyano,
- un groupe phényle, naphtyle, pyrryle, pyrimidyle, pyridyle, pyrrolidinyle ou morpholino qui peut être substitué quant à lui par du fluor, du chlore, du brome, un radical amino, hydroxy, nitro ou cyano, ou bien
- un groupe de formule -NR¹R² ou -OR⁴
n
- représente le nombre 0 ou 2,
R⁶
- est un groupe alkyle à chaîne droite ou ramifiée ayant jusqu'à 8 atomes de carbone, qui peut être substitué par du fluor, du chlore, du brome, un radical hydroxy, phényle ou un groupe de formule -NR¹R²,
- un groupe phényle qui peut être substituté par du fluor, du chlore, du brome, un radical hydroxy, cyano, nitro ou amino, ou
- un groupe de formule -NR¹R² lorsque n représente le nombre 2,
ou bien
D et E sont identiques ou différents et représentent
- un groupe de formules -NR¹R², -OR⁴ ou -COR⁵,
dans laquelle
R¹, R², R⁴ et R⁵ ont la définition indiquée ci-dessus,
D ou E forment conjointement avec B un noyau pentagonal à heptagonal saturé ou non saturé, qui est substitué le cas échéant par un radical alkyle linéaire ou ramifié ayant jusqu'à 6 atomes de carbone ou un radical phényle,
G
- est un atome d'oxygène ou un atome de soufre,
X
- est un groupe de formule -CH₂-CH₂- ou -CH=CH-
et
R
- est un groupe de formule 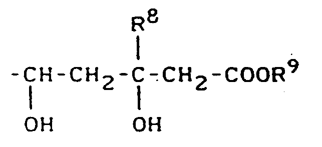 ou 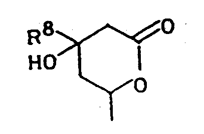
dans laquelle
R⁸
- représente l'hydrogène
R⁹
- est l'hydrogène ou un groupe alkyle linéaire ou ramifié, ayant jusqu'à 8 atomes de carbone ou un groupe benzyle, ou bien un groupe phényle ou un cation, ou bien
D
- représente le groupe de formule -X-R
dans laquelle
X et R ont la définition indiquée ci-dessus, et de leurs sels, caractérisé en ce qu'on réduit des cétones de formule générale (VIII) 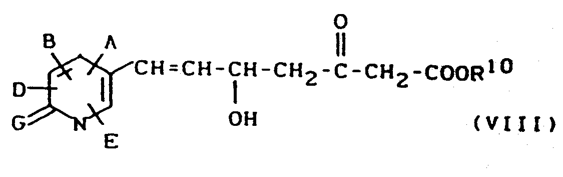 dans laquelle
A, B, D, E et G ont la définition indiquée ci-dessus,
et
R¹⁰ est un groupe alkyle,
dans le cas de la production des acides, on saponifie les esters,
dans le cas de la production des lactones, on cyclise les acides carboxyliques,
dans le cas de la production des sels, on saponifie ou bien les esters ou bien les lactones,
dans le cas de la production des composés éthyléniques (X = -CH₂-CH₂-), on hydrogène les composés éthéniques (X = -CH=CH-) par des procédés classiques, et on sépare éventuellement les isomères.

2. Procédé de production de cétones de formule 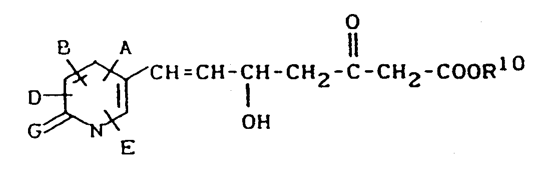 dans laquelle
A, B, D, E, G et R¹⁰ ont la définition indiquée dans la revendication 1,
caractérisé en ce qu'on fait réagir en présence de bases des aldéhydes de formule générale (IX) 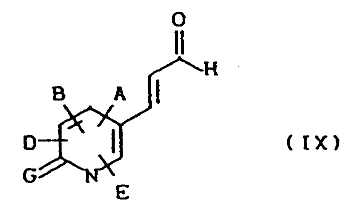 dans laquelle
A, B, D, E et G ont la définition indiquée ci-dessus,
dans des solvants inertes, avec un ester acétylacétique de formule générale (X) 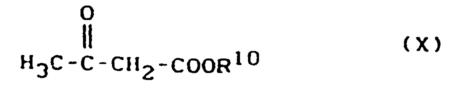 dans laquelle
R¹⁰ a la définition indiquée ci-dessus.
